# EUROPEAN PATENT SPECIFICATION

(11) **EP 4 371 537 B1**
(45) Date of publication and mention of the grant of the patent: **18.11.2026**
(21) Application number: 24167383.9
(22) Date of filing: 09.04.2018
(51) Int. Cl.: A61F 9/007

(54) **DEVICE FOR CREATING A CHANNEL IN SOFT TISSUE**
VORRICHTUNG ZUR ERZEUGUNG EINES KANALS IN WEICHGEWEBE
DISPOSITIF DE CRÉATION D'UN CANAL DANS UN TISSU MOU

(30) Priority: 09.04.2017 IL 25168417; 06.12.2017 US 201762595172 P
(43) Date of publication of application: 22.05.2024
(62) Document number(s) of the earlier application(s) in accordance with Art. 76 EPC: 18784603.5 / 3 609 447
(73) Proprietor: Sanoculis Ltd., 5555110 Kiryat Ono (IL); TEL HASHOMER MEDICAL RESEARCH INFRASTRUCTURE AND SERVICES LTD., 5262000 Ramat-Gan (IL)
(72) Inventor: LAVI, Gilad, 7574017 Rishon Le'zion (IL); GLOVINSKY, Yoseph, 4955047 Petah Tiqwa (IL); SHMUKLER, Vadim, 7529047 Rishon Le'Zion (IL); ISRAELI, Nir, 5290429 Ramat-Gan (IL)
(74) Representative: Zacco GmbH

(56) References cited:
- US-A- 4 002 169
- US-A- 5 487 725
- US-A1- 2009 112 119
- US-A1- 2015 011 926

## Description

### TECHNOLOGICAL FIELD

The present invention is in the medical field and relates specifically to surgical devices, and more specifically to miniature surgical cuttings tools.

### BACKGROUND

Removing tissue from the body is solicited in various scenarios including for diagnosis or treatment purposes. For example, in biopsy procedure, a sufficiently small tissue specimen is acquired in order to undergo examination outside the body. Usually, the shape of the specimen or the cavity left at the site of the removed tissue have low or no importance, the body heals from the injury leaving apparently no traces. In another example, tissue is removed in order to create paths for drainage of excessive liquids such as in Glaucoma condition (excessive intra ocular pressure).

Several surgical procedures are practiced to treat Glaucoma and/or elevated intraocular pressure (IOP). Filtering surgeries are used to gain access to the inner layers of the eye in order to create a drainage channel from the anterior chamber to the external surface of the eye under the conjunctiva, allowing aqueous humor to seep into a bleb from which it is slowly absorbed. Filtering surgeries are divided into either penetrating or non-penetrating types depending upon whether an intraoperative entry into the anterior chamber occurs. Scar formation at the site of operation may block aqueous humor circulation. Surgical adjuvants may be used to facilitate healthy tissue regeneration and keep created drainage channels functional.

Trephination to create *ab interno* sclerostomies was reported by Brown et al (Brown RH, Lynch MG, Denham DB, et al. Internal sclerectomy with an automated trephine for advanced glaucoma. Ophthalmology 1988; 95:728-734), and by SHIHADEH et al (Wisam A. Shihadeh, MD, Robert Ritch, MD, Jeffrey M. Liebmann,

MD. Rescue of failed filtering blebs with ab interno trephination. Cataract Refract Surg 2006; 32:918-922), as a way of performing filtering procedures, after failure of other procedures due to blocking, while maintaining the integrity of the overlying conjunctiva at the treated site.

US 4 002 169 A describes a method and an apparatus for performing surgery without tissue incision. According to this document, the introduction of surgical instruments into an animal body is effected, without tissue incision, by migrating a specially constructed hollow needle into the body behind a rotary augering tool journalled in the hollow needle. Surgical procedures may then be performed with the rotary tool or the same may be withdrawn from the in situ needle and different surgical implements substituted therefor which may be extended beyond the open end of the needle, as required. In the case of unwanted tissue removal, such as blood clots, cataracts and the like; a rotary masticating tool may be brought to the operative site by introduction through the hollow needle, the unwanted tissue masticated or liquefied and the same withdrawn through the needle bore following removal of the masticating tool. Similarly, fluids may be injected into the body and withdrawn therefrom, as required, through the hollow needle.

### GENERAL DESCRIPTION

The present invention is as defined by the claims.

The present disclosure provides a novel technique for removal of tissue from the body. The technique is particularly effective and useful in the controlled removal of soft tissue, e.g. by creating a well-defined, timely-controlled channel inside tissue. The technique also provides for verifying the channel's creation and its dimensions, without a need for using external verification techniques such as imaging, by retrieving and preserving the shape of the tissue removed. It should be noted that a "channel" as used herein means a pathway created in the tissue after removal of a corresponding tissue piece from the body. No parts, such as implant(s), are left in the body to create or maintain the integrity of the channel. Other expressions that are used interchangeably herein are "hole", "void" and "pathway". Specifically, the unique technique allows for controllably creating a channel in one or more adjacent target tissue layers being part of a multilayered tissue structure while preserving the one or more tissue layers covering/preceding and/or following the target tissue layer(s). Further, the unique technique provides the user with on-line feedback referring the success of the channel creating procedure by verifying the removed tissue volume and shape. The length and/or the diameter, of the removed tissue (e.g. having a cylindrical shape) matches/indicates about the length and/or diameter of the created channel. Minimizing the applied deformation on the removed tissue keeps it as close as possible to its original length and/or diameter, thereby providing improved and better real time feedback.

It is noted that the words "tissue layer" as used herein can mean a single tissue layer or a group of layers such as adjacent stacked layers (a multilayer) or a group of distinct layers. However, generally, the single tissue layer is the default meaning. Also, the "tissue layer" refers often to a tissue wall having a specific thickness and two sides (outer and inner, or proximal and distal) such that the channel/hole created therein extends between the two sides of the tissue wall.

For example, the channel may be a channel in the sclero-corneal junction of a subject's eye, which may be used to treat glaucoma by reducing intraocular pressure through providing fluid communication between the anterior chamber of the eye and the interface between the episclera and the conjunctiva tissues.

In the above-described literature (Brown et al and Shihadeh et al), the Ab interno trephination technique involve invasively introducing the surgical device into the anterior chamber of the eye through an incision made in the cornea opposite to the site of channel creation. This procedure is demanding, it depends heavily on the expertise of the surgeon and on the ability to accurately visualize the route of the surgical device inside the anterior chamber parallel to the iris into the filtration angle. It is also risky to vital organs such as the iris and lens as well as the angle structures that cannot be directly visualized unless an additional gonioscopic lens is used. This intraoperative gonioscopic lens is only rarely used even by glaucoma surgeons.

In contrast, the present disclosure provides a safe, minimally-invasive (*Ab externo*) and blazingly fast (in the order of seconds), highly-effective technique. As such, the present disclosure provides an opportunity for combined surgery by combining several surgical operations, for example combining treatment of high intraocular pressure according to the disclosure together with cataract surgery, thus saving time and effort from both the surgeon and the patient.

Although, as described above, the present disclosure is advantageous in its Ab externo application, the device of the present disclosure can also be safely and effectively applied in Ab interno procedure, because as will be further detailed below, the device includes an outer part that functions as a protector that is configured to protect organs of the eye, including internal organs, such as the iris, when utilized in Ab interno configuration.

At different sites in the body, the target tissue in which the channel has to be made underlies or precedes other tissue(s). In such a situation, the challenge is even greater because harm to the surrounding tissue(s) or adjacent should be avoided. One example is creating a channel in the sclera while keeping the conjunctiva intact. The medical device of the present disclosure is configured, in the Ab externo application, to optimize the remote penetration through the outer, first, tissue (e.g., conjunctiva) followed by cutting inner, second, tissue (e.g., sclera) to form a channel, while the minimal force possible is applied, such that the hole created in the upper tissue (conjunctiva) heals almost immediately leaving no traces. On the other hand, in the Ab interno application, the device, while being actually inserted into the anterior chamber of the eye, its construction and way of action ensure that no harm is caused to other organs, including the outer conjunctiva, while it creates a channel in the scelra tissue from inside.

Further, the medical device of the present disclosure is configured for easy automatic or semi-automatic operation, relieving burden from surgeon and providing him continuous feedback over the whole surgical procedure. The technique of the present disclosure aids the surgeon in safe positioning of the device inside the tissue to be cut while still allowing him/her control over the channel's three-dimensional orientation.

Moreover, the device of the present disclosure may incorporate authentication or validation features by retaining form/specimen of tissue removed from the body during the channel creation. The shape of the cutting tool of the device also provides enhanced trapping of the removed tissue within the cutting tool and prevents or minimizes the chances of leaving the removed tissue within the tissue wall such as the eye wall.

The channel created by the technique of the present disclosure is advantageous, for example in comparison to other techniques that leave implant(s) inside the tissue so to insure the drainage of fluid, because nothing is left in the tissue, except for the created void/hole/channel extending between the two side walls of the specific target tissue layer or multilayer, as the case may be. In other words, the created channel is a hole through the tissue with no artificial tube/shunt left inside the target tissue at all. Therefore, the created channel can be dynamic acting as a pressure regulator, i.e. it can regulate its drainage capacity by changing its size based on the pressure acting on its both ends. When the pressure gradient increases the channel opens/increases its size accordingly, and when the pressure gradient decreases the channel closes/decreases its size accordingly.

The range of sizes of the channel can be controlled by the geometry and size of the device that creates the channel. In the specific example of creating a channel in the eye wall, to treat elevated IOP for example, the present disclosure is advantageous in achieving the channel creation and verification in the micro scale, as the desired dimensions of the drainage channel are typically about 0.1-0.2mm in diameter and 1-1.5mm in length, supposing a substantially cylindrical shape for the channel and the matching removed tissue. The present disclosure accomplishes the targets above in the micro level while overcoming the limitations of the currently available techniques. Inter alia, available techniques may be used to produce tools having tissue receiving cavities of up to about 0.5mm length with the required above-mentioned diameter. However, this is not suitable for creating a substantially cylindrical channel within the eye wall that has a 1.5mm length. The technique of the present disclosure enables creating cutting tools with micro-scale desired dimensions in diameter and length, thereby enabling to preserve the shape of the removed tissue to be used for verification of the channel creation.

Generally, the medical device of the present disclosure is configured to operate in three distinct phases, a positioning phase characterized by an essentially linear advancement of the device along its linear longitudinal axis through one or more tissue layers until reaching the target tissue and stabilizing there inside the target tissue by an anchoring/sticking portion of an outer part of the device, a channeling phase during which an inner rotatable cutting tool of the device is rotated around its linear longitudinal rotation axis and then advanced to project from the outer part of the device and progress inside the target tissue to cut tissue of the target tissue and create the channel with the desired dimensions (diameter, cross-section area, length...), and a withdrawal phase in which the inner rotatable cutting tool is withdrawn from the target tissue into the outer part of the device and the whole device is retracted from the body. The withdrawal phase may be with or without rotation of the inner rotatable cutting tool depending, inter alia, on the tissue characteristics (kind, stiffness, region in the body), the time of operation and the desired channel shape. Typically, the outer part does not rotate during any of the phases and it only moves straight forwards and backwards on the linear longitudinal axis of the device. Generally, the outer part functions as a protective shaft, that protects the surrounding tissue during advancement of the device until reaching the target tissue, and as a stabilizing part such that its front (distal) portion is inserted /anchored/stuck in the target tissue to enable stable activation and performance of the inner rotatable cutting tool during the channeling phase.

Thus according to a broad aspect of the disclosure there is provided a medical device for removing a predetermined shape of soft tissue from a target tissue layer thereby leaving a matching channel with predetermined geometry and orientation between two side walls of the target tissue layer, the device comprises coaxial outer and inner elongated members extending along axis X;
said outer member comprises an open distal side and a first distal part configured for sticking to said target tissue layer (or multilayer), during forward axial movement;
said inner member comprises a second distal part configured to rotate and project distally through said open distal side to said predetermined shape of the soft tissue from the target tissue layer and create said channel formed as a hole through the target tissue layer or multilayer.

In some embodiments, the first distal part is configured for penetrating at least one other tissue layer preceding the target tissue layer during the forward axial movement.

In some embodiments, the first distal part comprises a tissue piercing tip at its distal end configured and operable to penetrate said at least one other tissue layer and said target tissue layers and a proximal portion at its proximal side configured and operable to penetrate said at least one other tissue layer and to stop at said target tissue layer, thereby sticking said outer member in the target tissue layer.

In some embodiments, said first distal part has a mid-portion between said tip and said proximal portion having a shape and an orientation that complement a shape and an orientation of said second tissue layer.

In some embodiments, the first distal part has a predefined length such that said tip does not exit distally from said target tissue layer.

In some embodiments, the proximal portion is a rim of said outer member, formed by cutting a section of wall of the outer member along said axis X.

In some embodiments, the inner member is fixedly attached to and housed within said outer member during said forward axial movement of the outer member.

In some embodiments, the outer member is manually moved during said forward axial movement until its said sticking in the second tissue layer.

In some embodiments, the inner member, while rotating, is manually moved along said axis X to create the channel.

In some embodiments, the device comprises a constant-force moving mechanism configured and operable to move said inner member, while rotating, along said axis X under a constant force. In some other embodiments, the device comprises a constant rate moving mechanism configured and operable to move said inner member, while rotating, along said axis X with a constant rate.

In some embodiments, the device comprises an electric motor configured and operable for axially moving and / or rotating said inner member.

In some embodiments, the device comprises a cavity for collecting tissue cut from said target tissue layer during creation of said channel. In some embodiments, the cavity is located within said inner member. In some embodiments, the cavity is located in a space between said inner and outer members.

In some embodiments, the second distal part of said inner member is open at its distal end and comprises a round cutting edge configured to attach to and cut soft tissue while rotating. The inner member may comprise a chamber for retaining a full shape of tissue cut from said second tissue layer during creation of said channel.

In some embodiments, the inner member comprises:
- an elongated round body extending along a longitudinal axis and having a uniform outer diameter at a proximal side thereof,
- a cutting portion at a distal side of the elongated body, comprising at a distal end thereof a round cutting edge of a first diameter being smaller than said outer diameter and a distally and continuously decreasing outer diameter, and
- a cavity extending along the longitudinal axis inside the cutting tool from said cutting portion, the cavity having dimensions matching said soft tissue shape,
wherein said tissue shape is cylindrical and has a length of about 1.5mm and a diameter of between about 0.1mm and about 0.2mm.

In some embodiments, the inner member comprises:
- an elongated round body extending along a longitudinal axis and having a uniform outer diameter at a proximal side thereof,
- a cutting portion at a distal side of the elongated body, comprising at a distal end thereof a round cutting edge of a first diameter being smaller than said outer diameter and a distally and continuously decreasing outer diameter, and
- a cavity extending along the longitudinal axis inside the cutting tool from said cutting portion, the cavity having a length of at least the length of the removed tissue,
wherein said cavity has a cavity diameter smaller than said first diameter at a distal end of the cavity and which increases continuously towards a proximal end of the cavity.

In some embodiments, the inner member comprises:
- an elongated round body extending along a longitudinal axis and having a uniform outer diameter at a proximal side thereof,
- a cutting portion at a distal side of the elongated body, comprising at a distal end thereof a round cutting edge of a first diameter being smaller than said outer diameter and a distally and continuously decreasing diameter, and
- a cavity extending along the longitudinal axis inside the cutting tool from said cutting portion, the cavity having a length of at least the length of the removed tissue,
wherein said cavity has a constant cavity diameter being equal to said first diameter, and wherein said first diameter is between about 0.1mm to about 0.2mm.

In some embodiments, the inner member comprises a tissue trapper comprising a slit formed in a wall of the body of the inner member along at least part of said cavity. In some embodiments, the slit is formed by tangential cutting of the wall of the body of the inner member, said device thereby further comprising an outer cavity located between the inner and outer members. In some embodiments, the slit is formed by radial cutting of the wall of the inner member.

In some embodiments, the second distal part of said inner member is configured as a drill bit configured for removing soft tissue.

In some embodiments, the rotating of said second distal part comprises clockwise and anti-clockwise reciprocal movement.

In some embodiments, the tissue piercing tip is configured as a lancet.

In some embodiments, the first distal part of the outer member is formed by cutting the outer member in the direction of the axis X along a curved line chosen to provide smooth penetration, at a distal segment of the first distal part, with increasing resistance-to-progression force, at a proximal segment of the first distal part.

In some embodiments, the at least one other tissue layer comprises the conjunctiva and / or the tenon and said target tissue layer is the episclera and/or the sclera and/or the cornea of an eye.

In some embodiments, the predetermined geometry of the channel is selected to enable pressure regulation of a treated eye over a predetermined time period.

According to yet another broad aspect of the disclosure there is provided a method for producing a cutting tool used in cutting soft tissue, the cutting tool comprising a distal cutting portion having at a distal end thereof a round cutting edge of a first diameter and a cavity extending for a predetermined length along a longitudinal axis of the cutting tool from said cutting portion and comprising a cavity diameter being either constant or increasing proximally along the predetermined length, the method comprising:
- providing a tool comprising at a distal side thereof a hollow cylinder having uniform outer and inner diameters and extending along at least said predetermined length, wherein said inner diameter is larger than said first diameter,
- shaping a distal portion of the hollow cylinder with a predetermined pattern such that both said inner and outer diameters decrease towards a distal end of the hollow cylinder and such that said first diameter is larger than said inner diameter and is smaller than said outer diameter at the distal end, and
- removing a slice of the hollow cylinder along said distal portion, such that the inner diameter at the distal end is substantially equal to said first diameter and the inner diameter at a proximal end of the distal portion is substantially equal to said cavity diameter.

In some embodiments, said shaping of the distal portion is carried out by swaging and/or spinning technique(s).

In some embodiments, said shaping of the distal portion is carried out by tapering technique.

In some embodiments, said predetermined pattern is linear.

In some embodiments, said predetermined pattern is non-linear.

In some embodiments, said cavity diameter is equal to said first diameter.

In some embodiments, the method further comprising: sharpening said round cutting edge from an internal side of the cutting portion, thereby providing that the cavity diameter at a proximal end of the cutting portion being smaller than the first diameter. In some embodiments, said cavity diameter increases proximally.

In some embodiments, the method further comprising coating an inner surface of said cavity with a friction-lowering composition.

In some embodiments, said predetermined length is at least 1.5mm.

In some embodiments, said cavity diameter at a proximal side of the cavity is between 0.1mm and 0.2mm.

In some embodiments, said uniform outer and inner diameters of the hollow cylinder are about 0.3mm and 0.16mm respectively.

In some embodiments, after shaping, said outer and inner diameters of the hollow cylinder at the distal end are about 0.27mm and 0.13mm respectively.

In some embodiments, said distal portion of the hollow cylinder has a length along the longitudinal axis of between about 1mm to about 2mm.

According to yet another aspect of the present disclosure there is provided a cutting tool for removing a predetermined shape of soft tissue while revolving and progressing, thereby leaving a matching channel between two walls of the soft tissue, the cutting tool being produced according to the method described above.

According to yet another broad aspect of the disclosure there is provided a method for creating a channel with predetermined geometry in a target tissue layer, the channel being formed as a hole extending between two side walls of the tissue layer, the method comprising:
providing a device comprising coaxial outer member comprising a first distal part configured for sticking to said tissue layer during forward axial movement and inner member comprising a second distal part configured to cut and remove tissue to thereby create said channel;
positioning said device at the target tissue layer by progressing the device along an axis X while said inner member is located inside said outer member until reaching said target tissue layer where the first distal part of the outer member is pushed into the target tissue such that at least a distal portion of the distal part is stuck inside the target tissue layer;
creating said channel by rotating and projecting said second distal part from said outer member to thereby cut and remove tissue from said target tissue layer and store the removed tissue in the device;
retracting said second distal part proximally out of said target tissue layer and into the inside of the outer member; and
pulling the first distal part proximally out of said target tissue layer and withdrawing the device out of the body substantially along the axis X.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Figs. 1A-1B** illustrate a non-limiting exemplary embodiment of a device
**Figs. 2A-2B** illustrate another non-limiting exemplary embodiment of a device
**Figs. 3A-3E** exemplify a non-limiting technique for creating a channel in soft tissue
**Figs. 3F-3I** exemplify another non-limiting technique for creating a channel in soft tissue
**Figs. 4A-4D** illustrate non-limiting examples of a part of a device
**Figs. 5A-5D3** illustrate non-limiting examples of a part of a device
**Figs. SE1-SE7** - illustrate one non-limiting scenario of creating a channel in soft tissue and specifically in the eye wall;
**Figs. 5F-5G4** illustrate non-limiting examples of a device and methods for producing the device
**Figs. 6A-6D** illustrate a non-limiting example of a manual movement mechanism
**Figs. 7A-7D** illustrate another non-limiting example of manual movement mechanism
**Figs. 8A-8D** illustrate yet another non-limiting example of manual movement mechanism
**Figs. 9A-9E** illustrate a non-limiting example of automatic movement mechanism and
**Figs. 10A-10D** illustrate another non-limiting example of automatic movement mechanism

### DETAILED DESCRIPTION OF EMBODIMENTS

The present disclosure provides a technique for creating a well-defined channel in a soft tissue. In one aspect, a medical device for removing a predetermined shape of soft tissue from a target tissue layer (or a first group of target tissue layers) thereby leaving a matching/corresponding channel with predetermined geometry and/or orientation through/between two side walls of the target tissue layer is provided. In some embodiments, such a device can be particularly useful in creating a drainage channel along the whole thickness of the episclera and/or sclera and/or cornea tissue (which will be generally referred to herein, for simplicity, as the sclera), of the eye to thereby treat excessive intraocular pressure. The sclera is covered by the conjunctiva and tenon tissues, such that approaching the sclera from outside requires penetrating the conjunctiva and the tenon. Therefore, the device may be also configured to penetrate through the conjunctiva/tenon before reaching the sclera.

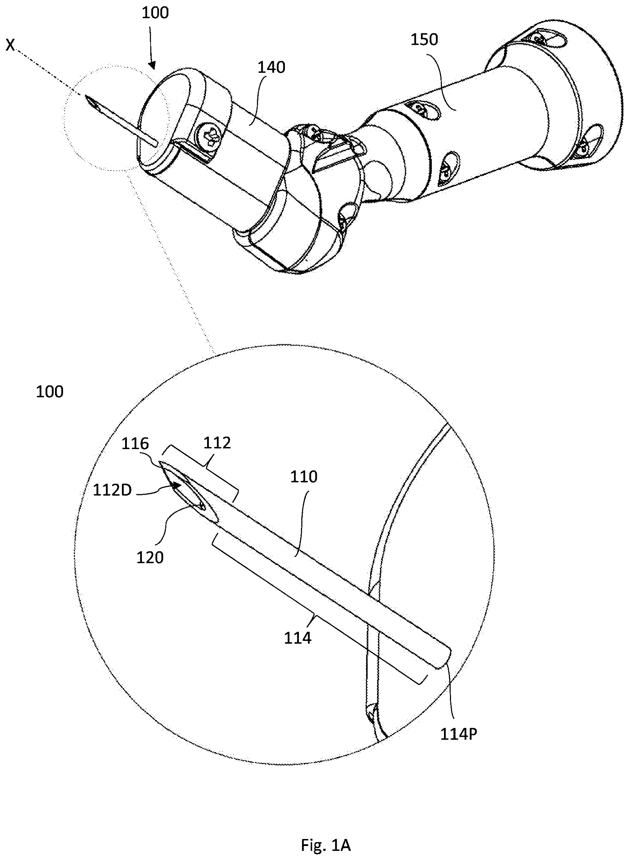

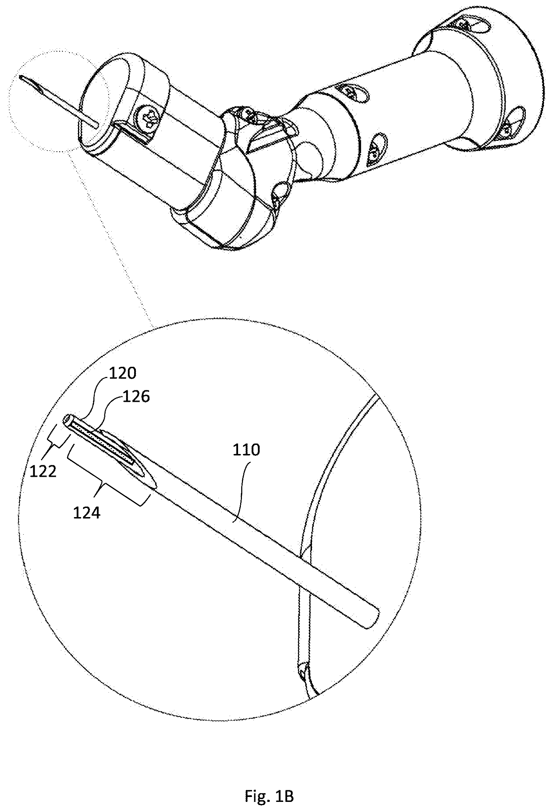

Reference is made to **Figs. 1A** and **1B** showing a specific, non-limiting, example of a medical device **100** according to some embodiments of the present disclosure.

The medical device **100** is configured for and capable of penetrating through first, upper, tissue layer(s) and creating a channel with predetermined geometry through a consecutive second, lower, target tissue layer. The device **100** includes coaxial outer and inner members, **110** and **120** respectively, extending along axis **X,** for creating the channel in the target tissue, and possibly penetrating the tissue layer(s) preceding the target tissue layer. The axis **X** is typically a longitudinal straight axis. The coaxial outer and inner members **110** and **120** are usually made from a hard, tough, material and are therefore rigid and do not bend when pushed/inserted/progressed through at least soft tissue. The coaxial outer and inner members **110** and **120** are mounted, at a proximal side **114P** thereof, on a handle/gripping unit **150** by which the user holds/grips the device **100** and operates it.

The outer member **110** includes an open distal side **112D,** a first distal part **112,** and a first proximal elongated part **114.** It is noted that the relative expressions "proximal" and "distal" as used herein, define relative orientation with respect to the user, such that" proximal" denotes the close side to the user and "distal" denotes the far side from the user. The outer member **110** is configured to move axially along the axis **X** to thereby penetrate soft tissue by its first distal part **112.** The axial movement of the outer member **110** is achieved by user manual operation. As it is manually operated by the user, the outer member **110** can be fixedly/firmly attached, at the proximal side **114P,** to the handle **150.** Alternatively, it can be configured for manual sliding by the user along axis **X** while not being firmly attached to the handle **150.** Details about the moving mechanism are described herein further below.

The first distal part **112** is configured for penetrating and passing through the tissue layer(s) preceding the target tissue layer, if any, during forward axial movement, and therefore it includes a tissue piercing tip **116,** at the distal end of the first distal part **112,** that enables the penetration. It is noted that, as the forward axial movement is manually controlled, the penetration of the preceding tissue layer(s), such as the relatively thin conjunctiva, is enabled by the manual pushing force applied by the user and which can be further facilitated by the manual lifting/pulling of the conjunctiva outwardly towards the user. The first distal part **112** is also configured to pierce the target, typically thicker, tissue layer, and stick into the target tissue layer so as to position the device inside the target tissue in which the channel is to be created, and provide the user with a pivotal point to define the three dimensional orientation of the channel. In addition to its plain name, the first distal part **112** is interchangeably called herein as "sticking part", "stabilizing part" or "anchoring part". It should be understood that while the first distal part **112** enters into the target tissue and sticks/anchors therein, it can be withdrawn backwardly by the application of a minimal force and without causing damage to the surrounding tissue. Sticking and/or anchoring as used herein do not mean a permanent state but rather a temporal, transitional state of the position of the first distal part, that gives the user a stable pivotal point of action.

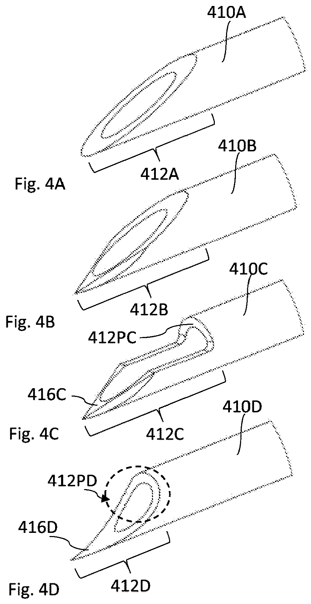

The tissue piercing tip **116,** formed at the most distal part of the first distal part **112,** can be configured according to the known in the art, e.g. as done with conventional medical needles. Accordingly, the tissue piercing tip **116** can include, for example, a beveled lancet structure. Yet, it can have other configurations, as will be further described below with reference to **Figs. 4A** to **4D****.**

The first proximal elongated part **114** is hollow, e.g. a hollow tube, enclosing and housing the inner member **120** there inside. Typically, the first proximal elongated part **114** has a cylindrical shape with a circular (round) or substantially circular transverse outer cross section. The first proximal elongated part **114** is configured to penetrate soft tissue smoothly and easily with minimum force, therefore it can have circular outer cross section and can be provided with a smooth (polished) outer surface to minimize friction during penetration into tissue. The inner cross section of the first proximal elongated part **114** is circular or has other shape that matches the outer surface of the inner member **120** enclosed therein.

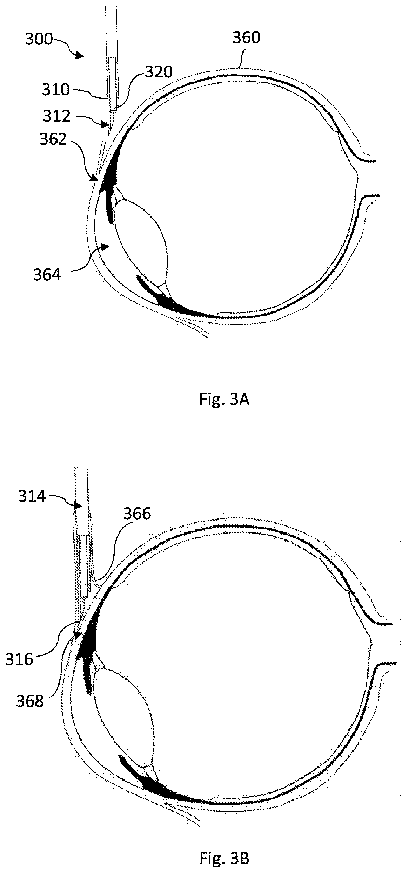

The inner member **120** includes a second distal part **122** and a second proximal elongated part **124.** The second distal part **122** is configured to project distally through the open distal side **112D,** approaching the target tissue while rotating, to thereby cut a predetermined shape of the target tissue and create the channel with the predetermined geometry and orientation in the target tissue, while the first distal part **112** is substantially positioned inside the target tissue as described above and as will be further exemplified below with reference to **Figs. 3A to 3I****.** In general, the second distal part **122,** at its distal end, is configured to provide effective attachment to the target tissue and to cut the target tissue while rotating. To this end, the distal end of the second distal part **122** can be provided with a cutting edge, a punching mechanism and the like, as will be further described below.

Generally, the device **100** includes a cavity/chamber **126** configured to collect the removed tissue therein, such that no tissue is left in the body. In some embodiments, the cavity/chamber is located inside the second proximal elongated part **124,** as exemplified in **Fig. 1B****.** In some other embodiments, the cavity/chamber **126** can be located in a space between the outer and inner members **110** and **120.**

The device **100,** including the handle **150** may be configured for single use, being disposable, therefore enhancing and maintaining safety and sterility of the device. The handle **150** can be configured as described in PCT/IL2016/051063 assigned to the assignee of the present invention.

The moving mechanism **140** is configured to enable axial movement of the outer member **110,** forwards (distally) and backwards (proximally), and both axial and rotational movement of the inner member **120.** The moving mechanism **140** can have manual (by the user) and/or automatic (by the use of mechanical and/or electrical means, such as a spring and/or a motor) operational modes for each of the movements it is capable of. The rotational movement of the inner member **120** can be in full or partial circles or rounds, clockwise and/or anticlockwise, and/or in reciprocal movement.

The construction and dimensions of the device can be costumed to match the application, the tissue properties, and anatomy and morphology of the site of body in which the channel is created.

For example, if used to create a drainage channel in the human eye, the dimensions of the device can be as follows:
The external diameter of the outer member is chosen to enable smooth and safe penetration into and withdrawal from tissue, while maintaining a minimal strength such that it does not break in the tissue during operation. It can be about 0.4 - 1.2 mm.

The overall length of the outer member is chosen to enable easy and safe access to the surgery site. It can be about 8 - 30 mm.

The length of the first distal part of the outer member can be chosen to enable insertion/sticking/anchoring of the first distal part into the second tissue, i.e. the sclera in this instance, while assuring that the first distal part does not protrude distally from the sclera, thus minimizing or cancelling invasive entrance into the anterior chamber of the eye. It can be about 0.5 - 3mm.

The external diameter of the inner member is chosen to create the predetermined geometry of the channel, while maintaining a minimal strength such that it does not break in the tissue during operation. It can be about 0.2 - 0.5 mm.

The overall length of the inner member is chosen to enable its connection to a moving mechanism at the proximal side while providing sufficient forward distance to create the desired channel length. It can be about 15- 40 mm.

The length of the second distal part of the inner member depends on the second distal part's specific construction that insures the channel creation.

During the channel creation, the inner member protrudes/projects from the outer member by about 1 - 4mm.

The inner member's rotation can be in the range of about 1 - 10,000 rpm. And, the penetration force is about 0.2 - 10 Newton.

The resulting channel's diameter would be about 0.1 - 0.5 mm.

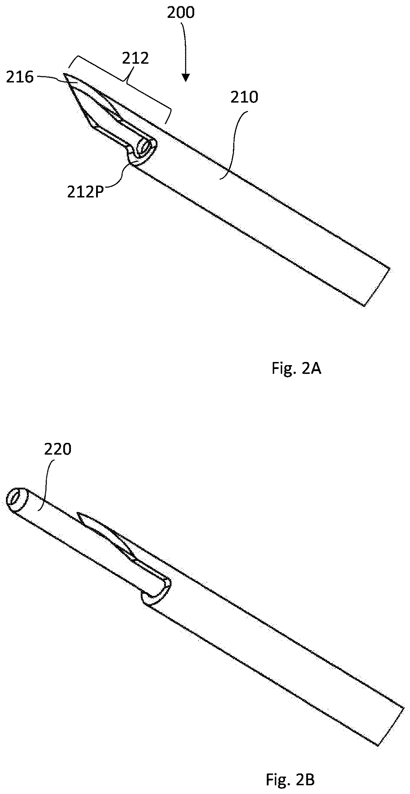

Reference is made to **Figs. 2A** and **2B****.** Throughout the text, functional parts which have the same functionality, have the same numbers with difference of one hundred duplicates. For example, the number **210** denotes an outer member and the number **220** denotes an inner member, both configured as at least having the features described above with respect to outer member **110** and inner member **120,** with possibly additional features. In the following various non-limiting embodiments of the device, including its outer and inner members and its moving mechanism will be exemplified. It should be understood, that any combination of one outer member, one inner member and one moving mechanism is equally possible.

**Figs. 2A** and **2B** exemplify a non-limiting example of the device **200** of the disclosure. In the figures, an outer member **210** and inner member **220** of the device **200** are shown. The outer member **210** and the inner member **220** are configured and operable at least as the outer member **110** and inner member **120** described above. **Fig. 2A** (as well as Fig **1A**) illustrates the device during the positioning phase, i.e. during inserting the device through the first and second (target) consecutive tissues, in which the outer member **210** leads the device into its position inside the second tissue to be channeled, and the inner member **220** (as well as 110 in Fig. 1A) is housed entirely in the outer member **210.** **Fig. 2B** (as well as **Fig. 1B**) illustrates the device during the channeling phase, i.e. during creation of the channel by the rotational and forward movement(s), projection, of the inner member **220.** As shown, the first distal part **212** of the outer member **210** includes a piercing portion / tip **216** at its most distal side, being configured as described above and operable to pierce and penetrate through tissue layer(s) preceding the target tissue, and to pierce, without fully penetrating, the target tissue layer. In addition, the first distal part **212** includes a portion **212P** at its proximal side configured and operable to pierce and penetrate the tissue layer(s) preceding the target tissue layer and to stop at the second (target) tissue layer, i.e. the portion **212P** prevents the outer member **210** from excessively penetrating the second (target) tissue in which the channel is created thereby sticking the outer member **210,** by its distal piercing portion **216,** in the target tissue layer. The portion **212P** is interchangeably called herein as "stopping portion" or "stopper".

In the described example, the stopper **212P** is an integral portion of the outer member **210** formed by a rim of the transverse, round, cross section of the outer member **210** by cutting a section of wall of the outer member **210** substantially along the axis **X.** Specifically, the section cut is a wall of the cylinder of the outer member **210,** e.g. half of the cylinder of the outer member between its most distal end and up to a proximal point along the outer member. The length of the wall section cut along axis **X** defines the length of the first distal portion **212** and the latter defines the extent of sticking the outer member **210** into the target tissue such that the distal end of the piercing tip **216** does not protrude/exit distally from the target tissue layer.

Reference is made to **Figs. 3A to 3I** exemplifying non-limiting techniques for creating a channel in soft tissue by using the medical device of the disclosure. The described example relates to creating a channel in the sclera tissue of the eye. However, as has already been said, the disclosure is not limited to this application and can be practiced at other regions in the body where creation of a controlled channel in a tissue layer preceding/beneath other tissue layer(s) is needed. Specifically, the disclosure enables channel creation at a region in the body which needs a clear and defined stopping/localization/stabilization feature of the device therein because the region cannot provide this feature; such a region is the soft tissue. The example described in **Figs. 3A to 3E** relates to Ab externo procedure where the device approaches the sclera tissue from outside. A human eye **360** is shown, where a channel should be created at the region of the sclera-corneal junction **362.** The created channel will controllably connect between the anterior chamber **364** of the eye and the sub conjunctival space/zone and hence allow the extra fluid accumulated in the anterior chamber to exit and by this reduce the intraocular pressure. As has been described earlier, the channel size can be controlled by providing a device with specific geometrical dimensions. Also, when used for treating excessive pressure, the size of the created channel is determined based on the magnitude of excessive pressure that should be treated. Higher pressure requires bigger channel and vice versa. The created channel insures effective regulation of pressure such that it expands or contracts between controlled sizes based on the pressure gradient across the channel, i.e. the pressure difference between inside and outside the eye.

As shown in **Fig. 3A****,** the device **300** approaches the eye from outside where it encounters the outer tissue layer that includes the conjunctiva and/or tenon tissues (**366** in **Fig. 3B**) by the outer member **310,** and precisely by the first distal part **312** of the outer member **310.** The outer member **310** pierces and penetrates the conjunctiva and/or tenon when it is advanced forwardly, typically manually, by the surgeon.

As shown in **Fig. 3B****,** after or while the device passes the conjunctiva and tenon **366,** the surgeon can pull the conjunctiva **366,** and possibly also the tenon, outwardly by the help of a suitable tool held in his other hand. The conjunctiva tissue, and possibly also the tenon, now wraps the outer member **310** at the first proximal elongated part **314.** This saves the conjunctiva and/or tenon by preventing them from coming into contact with the inner member which will be rotated and advanced to cut and remove sclera tissue. The tissue piercing tip **316** of the outer member **310** now contacts the sclera tissue **368.**

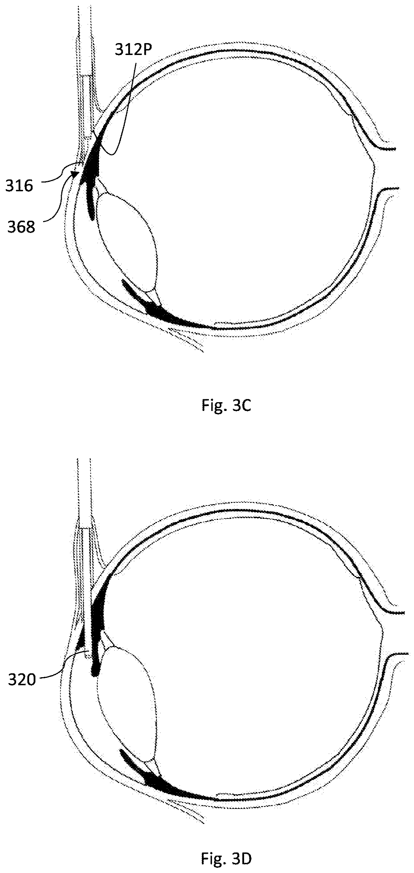

As shown in **Fig. 3C****,** the outer member **310** is further advanced forwardly, manually, as described above, such that the piercing tip **316** penetrates the sclera tissue **368.** The device, by the first distal part **312** of its outer member **310,** is stuck in (anchored) and stabilized temporarily in the sclera tissue **368.** During advancement inside the sclera tissue **368,** the resistance-to-progression increases and is given as a feedback to the surgeon when he/she manually advances the device. In the case the device is configured with the stopper **312P,** as shown in this specific example, the device **300** comes to a hard stop because the stopper **312P** provides significant increase in resistance-to-progression force on the outer member **310** and prevents the additional penetration/progression inside the sclera tissue **368.**

It is appreciated that **Figs. 3A** to **3C** illustrate the positioning phase of the device **300** as a preparation for the channeling phase. It is also appreciated, that during the positioning phase, no relative motion between the outer and inner member occurs. Generally, the inner member is hidden inside and fixedly attached to the outer member during the axial movement of the outer member, no matter how the axial movement of the outer member is executed, whether the axial movement includes manual displacement of the outer member by the surgeon relative to the handle, or whether the outer member is fixedly attached to the handle such that the axial movement of the outer member is generated by manual axial movement of the handle by the surgeon.

At this point, as shown in **Fig. 3D** and while the outer member **310** protects the conjunctiva tissue, the inner member **320** is rotated, either mechanically or electrically by a dedicated motor as described above and will be further exemplified below, and is advanced by the applied moving mechanism forwardly such that it contacts and attaches to the sclera tissue **368** and starts with drilling and creating the channel. The advancement distance of the rotating inner member can be configured by the moving mechanism such that the distal end of the inner member **320** does not protrude significantly into the anterior chamber of the eye to avoid causing harm to the internal side of the eye. The inner member **320** is then retracted backwardly (not shown), either rotating or not depending on its configuration, as will be described further below, until it is back in its secured position inside the outer member **310,** and the latter is pulled out from the sclera and conjunctiva tissues by the surgeon. The conjunctival tissue recovers almost immediately and the hole therein, formed by the outer member only, closes. Moreover, as during the positioning phase the surgeon pulls the conjunctiva outwardly, then after releasing the conjunctiva the hole in the conjunctiva will be displaced with respect to the channel in the sclera. As the conjunctiva attaches back to the sclera, the risk of eye collapse due to excessive fluid exiting the eye is prevented.

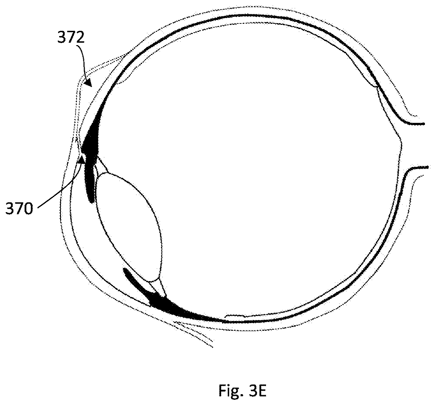

**Fig. 3E** illustrates the created channel **370** after the device is pulled outside the eye. The aqueous humor (the fluid in the anterior chamber) starts to exit the anterior chamber towards the sub-conjunctival space, such that a bleb **372** is formed under the conjunctiva and above the sclera, and the fluid is reabsorbed in the blood vessels at the vicinity thereof.

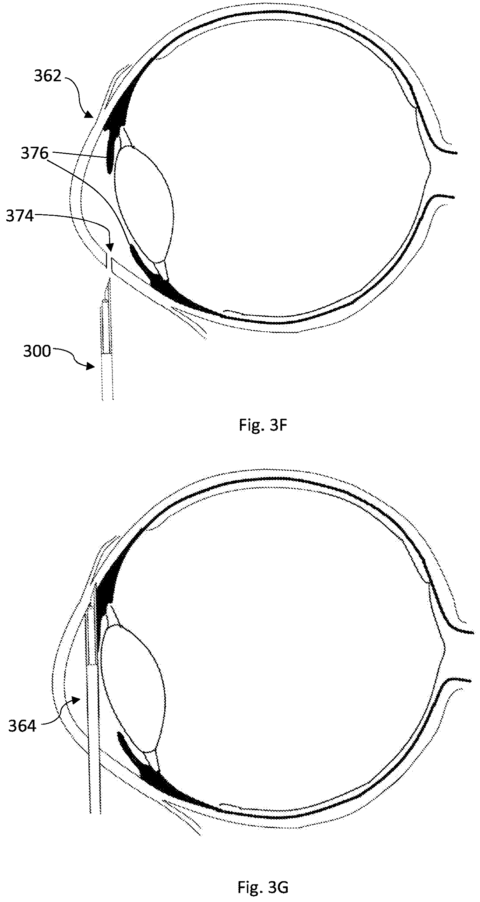

Reference is now made to **Figs. 3F to 3I** exemplifying another non-limiting technique for creating a channel in soft tissue by using the medical device of the disclosure.

The described example relates to creating a channel in the sclera tissue of the eye in Ab interno procedure by approaching the sclera tissue from inside of the eye. As has been mentioned above, the device of the disclosure is advantageous in that it can be used in either Ab externo or Ab interno procedures. For simplicity of presentation, every feature which is not referenced in the figures is assumed to be the same as in Figs. 3A to 3E. The human eye is shown, where a channel should be created at the region of the sclera-corneal junction **362,** as depicted in **Fig. 3F****.** As described above, the created channel will controllably connect between the anterior chamber of the eye and the sub conjunctival space/zone and hence allow the extra fluid accumulated in the anterior chamber to exit and by this reduce the intraocular pressure. The properties of the channel, including its size and geometry, can be as has been described earlier with reference to Figs. 3A to 3E. As shown in **Fig. 3F****,** the device **300** approaches the eye from outside and is to be inserted into the anterior chamber **364** of the eye through an opening **374** created beforehand in clear cornea at the opposite side to where the channel is to be created. The opening **374** can be achieved by conventional means known in the art such as a stylet blade. The device is inserted with respect to the eye in an orientation which is opposite to the orientation described in Figs. 3A to 3E relating to the Ab externo procedure. In other words, the tissue sharp tip of the outer member is now closer to the internal side of the eye, whereas it was farther away in the Ab externo procedure (as shown in Fig. 3A). By this, the first distal portion beveled shape and orientation will complement the shape and orientation of the sclera at the contact region **362.**

The device is inserted into the anterior chamber and is pushed manually by the surgeon, while passing above the iris **376,** until it contacts the sclera tissue at the sclera-corneal junction **362** from inside.

As appreciated from **Fig. 3G****,** after the surgeon feels the contact, another pushing force is applied manually in the forward direction, such that the outer member **310** pierces and penetrates the sclera (from the inside). The device, by the first distal part **312** of its outer member **310,** is stuck in (anchored) and stabilized temporarily in the sclera tissue. As described above, during advancement inside the sclera tissue, the resistance-to-progression increases and is given as a feedback to the surgeon when he/she manually advances the device. In the case the device is configured with the stopper **312P,** as shown in this specific example, the device comes to a hard stop because the stopper provides significant increase in resistance-to-progression force on the outer member and prevents the additional penetration/progression inside the sclera tissue. As also described above, the preconfigured length of the first distal portion of the outer member insures that the piercing tip does not exit the sclera from the other (here external) side, such that the conjunctiva or other covering tissue is not torn or pierced by the outer member.

It is appreciated that **Figs. 3F** and **3G** illustrate the positioning phase of the device 300 as a preparation for the channeling phase. It is also appreciated, that during the positioning phase, no relative motion between the outer and inner member occurs. Generally, the inner member is hidden inside and fixedly attached to the outer member during the axial movement of the outer member, no matter how the axial movement of the outer member is executed, whether the axial movement includes manual displacement of the outer member by the surgeon relative to the handle, or whether the outer member is fixedly attached to the handle such that the axial movement of the outer member is generated by manual axial movement of the handle by the surgeon.

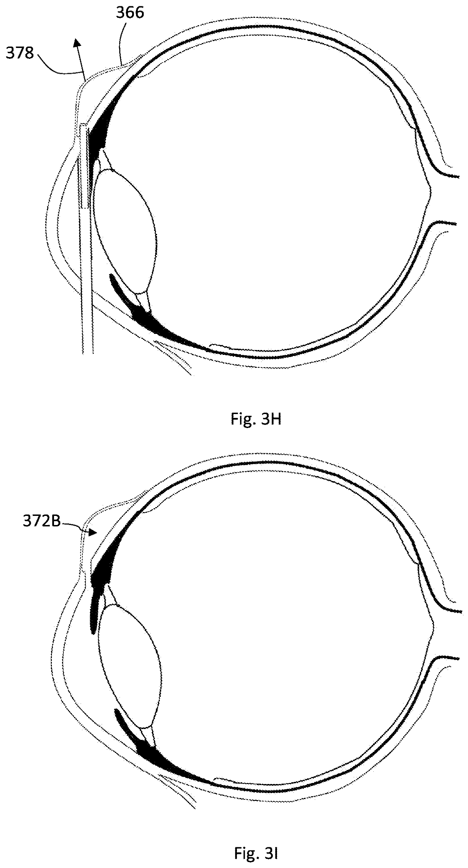

As shown in **Fig. 3H****,** while or after the outer member is anchored into the sclera tissue, the surgeon can pull and lift the conjunctiva **366,** and possibly also the tenon, outwardly in a direction **378** by the help of a suitable tool held in his other hand. This saves the conjunctiva and/or tenon by preventing them from coming into contact with the inner member which will be rotated and advanced to cut and remove sclera tissue. The inner member is rotated, either mechanically or electrically by a dedicated motor as described above and is advanced by the applied moving mechanism forwardly such that it contacts and attaches to the sclera tissue and starts with drilling and creating the channel. The advancement distance of the rotating inner member can be configured by the moving mechanism such that the distal end of the inner member does not protrude significantly outside the sclera tissue to avoid causing harm to the conjunctiva and/or tenon tissues. While the inner member rotates to cut and remove tissue from the sclera, the outer member, which is stabilized by its anchor to the sclera tissue, is stationary, it does not or hardly moves, thus preserving the internal organs, such as the iris, from any damage that may have been caused by the rotating inner member. Further, its anchoring to the sclera minimizes any accidental pulling of the rotating inner member from the sclera, something which may otherwise have adverse consequences on the internal organs of the eye. After creating the channel, the inner member is retracted backwardly (not shown), either rotating or not depending on its configuration, as will be described further below, until it is back in its secured position inside the outer member, and the device is pulled backwardly out from the anterior chamber and out of the eye through the opening **374** which can be treated by suitable medicines in order to heal and close almost immediately.

**Fig. 3I** illustrates the created channel after the device is pulled outside the eye. The aqueous humor (the fluid in the anterior chamber) starts to exit the anterior chamber towards the sub-conjunctival space, such that a bleb **372B** is formed under the conjunctiva and above the sclera, and the fluid is reabsorbed in the blood vessels at the vicinity thereof.

Reference is made to **Figs. 4A** to **4D** showing various non-limiting examples of the outer member of the device according to some non-limiting embodiments of the disclosure.

The figures are illustrative only and are not presented in a full scale. Specifically, the figures show different non-limiting configurations of the first distal portion of the outer member. Generally, the shape and/or orientation of the first distal part can be chosen to complement the shape and/or orientation of the target tissue, such that better coupling/attachment/adherence/anchoring between the outer member and the target tissue is achieved.

**Fig. 4A** shows a known shape of cannula end used in medical needles. This is a common point cannula end known as flat bevel point. This configuration can be used as the first distal part **412A** of the outer member **410A.**

**Fig. 4B** also shows a known shape of cannula end used in medical needles. This is a common point cannula end known as lancet bevel point. This configuration can be used as the first distal part **412B** of the outer member **410B.**

**Fig. 4C** shows a special non-limiting example of a first distal part **412C** of an outer member **410C** according to the disclosure. Similar configuration to **Fig. 4C** is also shown in **Figs. 2A** and **2B****.** The first distal part **412C** includes a tissue piercing tip **416C** configured as a lancet bevel point and a stopping portion **412PC** formed by the rim of the outer member **410C** which is obtained by cutting a section of the wall of the outer member **410C** along its longitudinal axis. In some exemplary, non-limiting, embodiments, half of the wall (e.g. half of cylinder) is cut.

**Fig. 4D** shows another special non-limiting example of a first distal part **412D** of an outer member **410D** according to the disclosure. The first distal part **412D** includes a tissue piercing tip **416D** and a stopping portion **412PD** both obtained by cutting the outer member **410D** in the direction of the longitudinal axis along a curved line. The curved line can be chosen to provide smooth transition along the first distal part enabling smooth penetration with increasing resistance-to progression force. The curved line is usually configured as a smooth, continuous line with constant or variable slope (its derivative trend is always positive or always negative, though not necessarily constant), although other non-continuous behavior can be used. For example, the curved line can be a combination of two or more line segments, among which some are curved and/or straight. Specifically, the piercing portion can be configured as a curved line while the stopping portion can be configured as a straight line, e.g. in the direction of the transverse cross section of the inner member. In some embodiments, such a smooth curved line can follow a circular, elliptical, semi- circular or semi-elliptical path, e.g. can be part of a circle's or an ellipse's circumference. In the example shown, an elliptical curve is presented, such that the elliptical major axis lies in the direction of the longitudinal axis of the outer member and the elliptical minor axis is in the orthogonal direction (across the outer member). In this instance, the major axis defines the length of the first distal part, and the minor axis (or, more specifically the relation between major and minor axes) defines the level of resistance-to-progression of the stopping portion **412PD.** It should be understood, that the above-described example relates to formation of the curved line, forming the piercing tip / stopper, along the longitudinal axis direction, from a single direction (2D forming), while any other shaping combination in 3D is also possible.

As has been clarified above, any configuration of the outer member can be used with any configuration of the inner member.

The inner member is configured, as described above, for attaching effectively to the second tissue (in which the channel is formed) and for cutting a well-defined geometrical shape of the tissue, both while rotating and advancing distally. In some embodiments, the inner member is configured for storing the cut tissue in its intact form, thus providing a validation and authentication to the created channel. In addition, storing the cut tissue inside the inner member (in the second proximal elongated part) serves in protecting the eye from sudden collapse by blocking the outflow of aqueous humor from the anterior chamber during the channel creation and/or when the device is pulled outwardly from the eye.

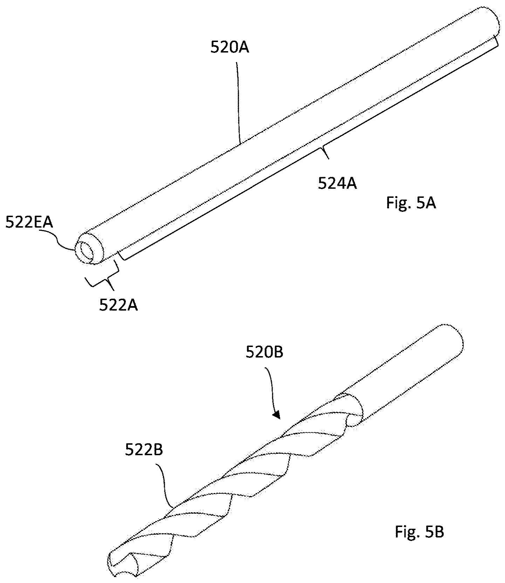

Reference is made to **Figs. 5A-5D** showing non-limiting examples of the inner member of the device according to some non-limiting exemplary embodiments of the disclosure.

The different examples can be distinguished by the specific channel creating application, including the specific dimensions of the channel which is affected by its purpose and its location in the body. Specifically, some of the described examples may be more suitable than others for the application of creating a channel in the eye wall for treating elevated IOP.

**Fig. 5A** illustrates an inner member **520A** having a second distal part **522A** configured to attach to tissue and cut tissue, while rotating and progressing distally, and to guide the inner member through the tissue, e.g. towards the anterior chamber of the eye. The inner member also includes a second proximal elongated part **524A** that includes an elongated chamber/cavity (not shown) configured to receive therein the tissue being removed. The outer diameter of the inner member should preferably match the inner diameter of the outer member such that no space is left there between. The shape of the chamber/cavity preferably matches the shape of the cut tissue. In action, the inner member approaches the tissue while rotating (at least the second distal part), so that the rotation creates desired attachment of the inner member to the tissue and enables start of the piercing and cutting. Generally, the second distal part **522A** has at the distal end a round cutting edge **522EA,** typically of a circular shape, having one of the following configurations:
- the round cutting edge **522EA** has a diameter equal to the diameter of the elongated cavity, such that the cutting edge is created by sharpening (grinding) in the direction from the outer diameter of the inner member towards the diameter of the elongated cavity;
- the round cutting edge **522EA** has a diameter equal to the outer diameter of the inner member, such that the cutting edge is created by sharpening in the direction from the diameter of the elongated cavity towards the outer diameter of the inner member; and
- the round cutting edge **522EA** has a diameter bigger than the diameter of the elongated cavity and smaller than the outer diameter of the inner member, such that the cutting edge is created by sharpening in both directions, from the outer diameter of the inner member towards the diameter of the elongated cavity and from the diameter of the elongated cavity towards the outer diameter of the inner member.

It was found by the inventors that the degree of sharpening, i.e. the inclination angle, plays an important role in providing effectively desired piercing of and/or attachment to the tissue.

**Fig. 5B** illustrates another non-limiting example of the inner member **520B.** In this example, the inner member is configured as a full-bodied, not hollow, elongated member with a second distal part **522B** configured as a drill bit being provided with a flute enabling creating the desired channel in soft tissue while rotating. The length, spiral, point angle and lip angle of the drill bit can all be adjusted for optimal soft tissue removal. In this instance, the inner member **522B** rotates with full rounds clockwise or anticlockwise, depending on the spiral direction, such that the removed tissue is conveyed backwardly far from the target tissue and towards a collecting cavity located between the inner member and the outer member of the device.

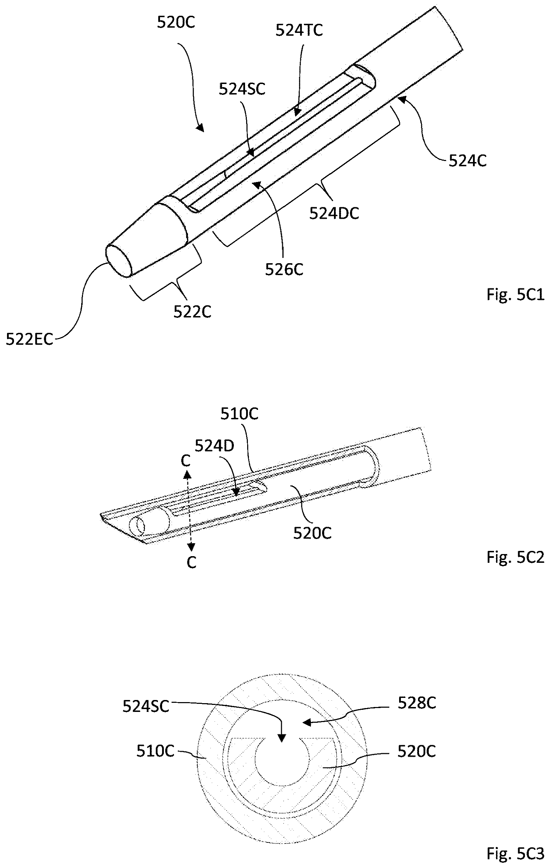

**Figs. 5C1-5C3** illustrate another non-limiting example of the inner member **520C.** **Fig. 5C1** is an isometric view of the inner member **520C.** **Fig. 5C2** is an isometric view of the coaxial outer and members, **510C** and **520C,** with half of the wall of the outer member at the distal side removed for easy illustration. **Fig. 5C3** illustrates a transverse cross section of the outer and inner members made along the line **C-C** in **Fig. 5C2****.** In this example, the inner member is configured partially similar to the example of Fig. 5A in that, as shown, the inner member **520C** has a second distal part **522C** configured to attach to tissue and cut tissue by its cutting edge **522EC,** while rotating and progressing distally, and to guide the inner member through the tissue. The inner member **520C** also includes a second proximal elongated part **524C** that includes an elongated chamber/cavity **526C** (inside the second proximal elongated part **524C,** shown in **Fig. 5C3**) configured to receive therein the tissue being removed. As also shown, the second proximal elongated part **524C** of the inner member **520C** includes a tissue trapper **524TC** at a distal segment **524DC** of the second proximal elongated part **524C,** located substantially in parallel to the elongated cavity **526C.** The tissue trapper / tissue-trapping enhancer **524TC** enhances and contributes to the trapping of the removed tissue during its removal, such that it allows/ensures pulling the removed tissue out of the body. In addition, the tissue trapper **524TC** can facilitate the flow of the removed tissue into the cavity **526C** by minimizing issues of clogging. In some embodiments, additionally or alternatively, the cavity in the inner member of the device can be designed to trap or contribute to trapping of the tissue there inside. In this example, the tissue trapper **524TC** includes a slit **524SC,** located in the longitudinal direction, i.e. along at least part of the cavity **526C.** The slit **524SC** is obtained by tangential cutting of the round wall of the inner member along the distal segment **524DC,** i.e. by cutting in the tangential direction to the inner member's wall/circumference. It should be noted that generally the tissue trapper **524TC** can include more than one slit along the inner member circumference, each slit being formed by tangential cutting along the longitudinal axis. **Fig. 5C2** illustrates the device either during the positioning phase or after the device has been pulled out of the body, while in both cases the inner member (and the removed tissue in the after operation case) is located safely inside the outer member. As shown in **Figs. 5C2 and 5C3** the tangential cutting of the inner member's wall forms, in addition to the slit **524SC,** a depression **524D** along the distal segment **524DC** of the inner member. The depression **524D** causes the formation of a second outer cavity **528C** between the inner and outer members, that may enhance the pulling of the removed tissue towards the cavity **526C** and/or the inside of the outer member **510C.** In other words, the depression **524D,** resulting from the tangential cutting, forms part of the tissue trapper **524TC.**

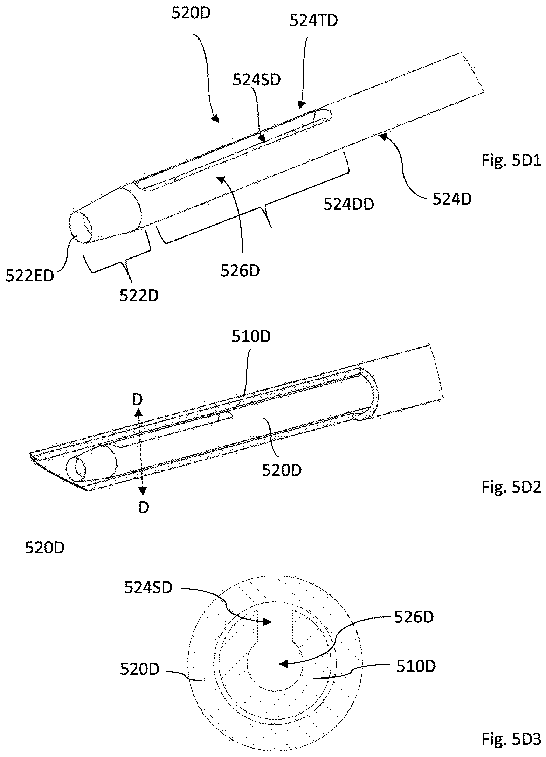

**Figs. 5D1-5D3** illustrate another non-limiting example of the inner member **520D** including a tissue trapper / tissue-trapping enhancer **524TD. Fig. 5D1** is an isometric view of the inner member **520D.** **Fig. 5D2** is an isometric view of the coaxial outer and members, **510D** and **520D,** with half of the wall of the outer member in the distal side removed for easy illustration. **Fig. 5D3** illustrates a transverse cross section of the outer and inner members made along the line **D-D** in **Fig. 5D2****.** As can be appreciated, various features and elements in **Figs. 5D1-5D3** are similar to those in **Figs. 5C1-5C3****.** Specifically, as shown in **Fig. 5D1****,** the inner member **520D** has a second distal part **522D** configured to attach to tissue and cut tissue by its cutting edge **522ED,** while rotating and progressing distally, and to guide the inner member through the tissue. The inner member **520D** also includes a second proximal elongated part **524D** that includes an elongated chamber/cavity **526D** (inside the second proximal elongated part **524D,** as shown in **Fig. 5D3**) configured to receive therein the tissue being removed. The second proximal elongated part **524D** of the inner member **520D** includes a tissue trapper **524TD** at a distal segment **524DD** of the second proximal elongated part **524D,** located substantially in parallel to the elongated cavity **526D.** As has been explained, the tissue trapper **524TD** enhances and contributes to the trapping of the removed tissue during its removal, such that it allows/ensures pulling the removed tissue out of the body. In addition, the tissue trapper **524TD** can facilitate the flow of the removed tissue into the cavity **526D** by minimizing issues of clogging. In some embodiments, additionally or alternatively, the cavity in the inner member of the device can be designed to trap or contribute to trapping of the tissue there inside. In this example, the tissue trapper **524TD** includes a slit **524SD,** located in the longitudinal direction, i.e. along at least part of the cavity **526D.** The slit **524SD** is obtained by radial cutting of the round wall of the inner member along the distal segment **524DD,** i.e. by cutting in the radial direction of the inner member. It should be noted that generally the tissue trapper **524TD** can include more than one slit along the inner member's circumference, each slit being formed by radial cutting in the radial direction and along the longitudinal axis. **Fig. 5D2** illustrates the device either during the positioning phase or after the device has been pulled out of the body, while in both cases the inner member (and the removed tissue in the after operation case) is located safely inside the outer member.

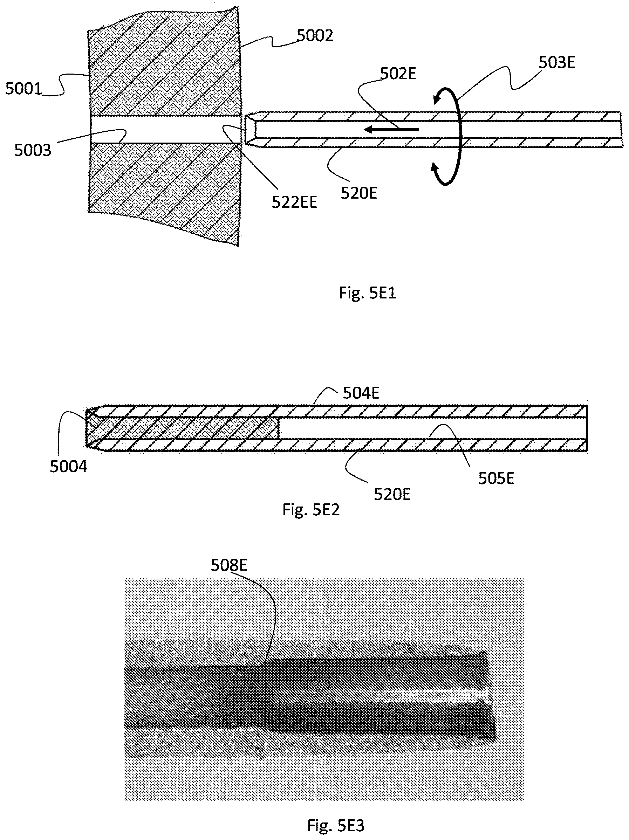

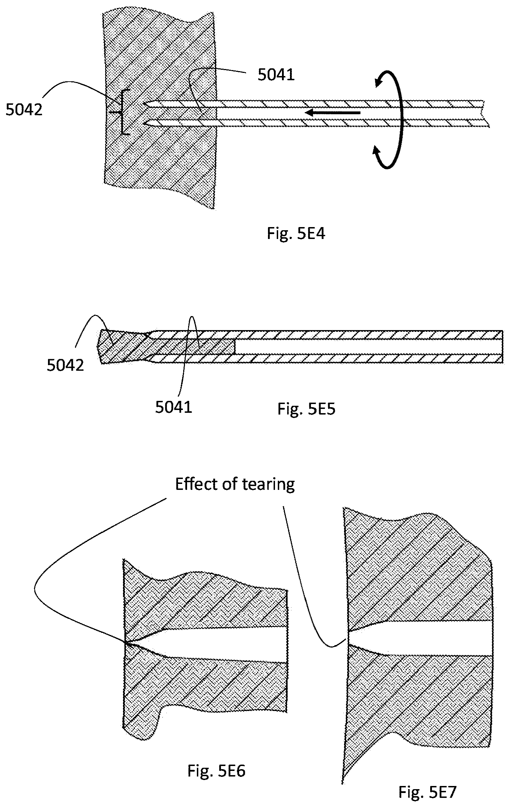

Turning now to **Figs. 5E1 to 5E7** illustrating one non-limiting scenario of removing soft tissue from a tissue layer in the body. Specifically, the figures illustrate undesired effect of tearing of the soft tissue, instead of or in addition to cutting, while rotating the cutting tool inside the tissue.

Ideally, the channel created in the tissue can be expected to look as shown in **Fig. 5E1****,** i.e. it should have a cylindrical shape, such as when the inner member is as described in Fig. 5A. The channel **5003** connects between outer scleral surface **5002** and inner scleral surface **5001.** The cutting tool **520E,** e.g. the inner member, rotates in a direction **503E** around its longitudinal axis, either clockwise or anticlockwise or reciprocating in both directions, and approaches the sclera under feeding rate **502E.** In preferable scenario, the channel should have the required dimensions while its diameter is similar to the diameter of the cutting edge **522EE.** The removed tissue **5004** is expected to be trapped within the cutting tool **520E** as shown in **Fig. 5E2****.**

It is appreciated that cutting of the tissue is defined by the tissue behavior and characteristics. As the cutting tool **520E** cuts, it rotates/revolves within the tissue. The treated organ (e.g. - the eye) is static while the cutting tool **520E** rotates/turns. The cutting tool **520E** presses the tissue both by its external surface **504E** (outer diameter) and inner diameter **505E.** The diameter of the cylindrical tissue **5004** is defined by the cutting edge **522EE** yet the inner diameter 505E might be slightly smaller and causes squeezing of the tissue (within the cavity of the cutting tool). Another reason for squeezing of the tissue inside the cavity of the cutting tool can be a relatively high friction force between the tissue and the inner surface of the cavity. Additional reason for squeezing of the tissue inside the cavity might be the limited length of the cavity as shown in **Fig. 5E3** which is a magnified image of a cutting tool, captured by a microscope. The current technology enables creating a cavity with a small diameter, as required in the treatment of the eye, with length up to about 0.5mm, as illustrated by the step **508E** in the figure. However, the required length for the channel may be longer than that, for example it should be about three times more (1.5mm) when the channel is to be created in the sclera in the eye wall.

Since the cutting tool 1(e.g. the inner member) rotates and the tissue (e.g., the eye) is static, the tissue **5004** is expected to remain static until the cutting process is completed. In reality, during the cutting process, as shown in **Figs. 5E4** and **5E5,** the tissue **5004** is defined by two sections, tissue section **5041** pressed into the cavity of the cutting tool and tissue section **5042** still un-pressed. The attachment of tissue section **5041** to the inner surface of the cavity, due to high friction or due to insufficient cavity length, may cause tissue section **5041** to start rotating with the cutting tool and to tear apart from tissue section **5042.** In this case, the separation of tissue **5004** is not caused by cutting, but rather by torsional tearing. Accordingly, the channel created within the eye wall might look as shown in **Figs. 5E6** or **5E7.** This may result in insufficient and ineffective drainage, or even no drainage at all.

Minimizing the radial attachment of the removed tissue to the inner surface of the cavity in the cutting tool enables continuation of cutting rather than tearing. Reducing the radial attachment force between the removed tissue (e.g., tissue 5041) and inner surface of the cavity (e.g., surface 505E) can be achieved by lowering the friction coefficient between the tissue and the inner surface of the cavity (for example by applying low friction coating on the inner surface). Alternatively or additionally, Reducing the radial attachment between the removed tissue and inner surface of the cavity can be achieved by creating specific geometry of the cutting tool, e.g. by making the diameter of the inner surface of the cavity bigger than the diameter of the cutting edge of the cutting tool.

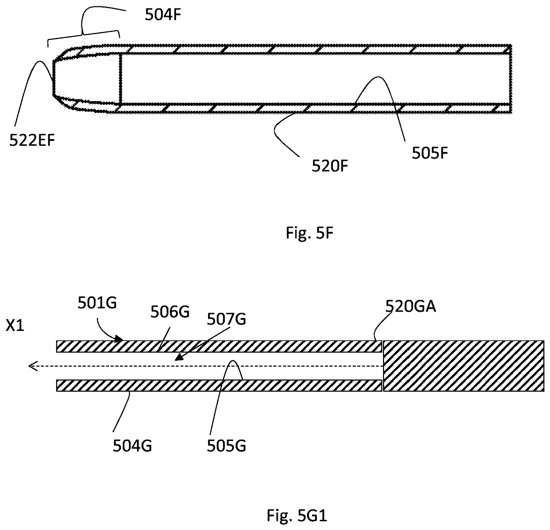

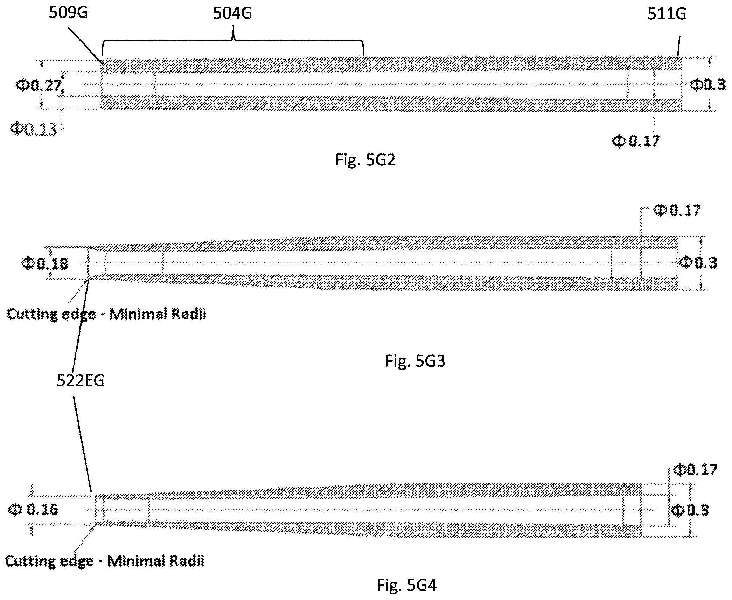

Turning now to Figs. **5F-5G** showing non-limiting exemplary embodiments of tissue cutting tools and methods of fabrication and/or optimization. The cutting tools are optimized for cutting soft tissue and for creating a channel with predetermined dimensions and geometry in a specific given tissue while minimizing the effect of tearing. In some embodiments, the inner member of the device of the disclosure can be configured as the cutting tools described in Figs. 5F and 5G. Therefore, the reference numbers used in the following figures follow the same numbering used so far, for example 520E denotes a tissue cutting tool that can be used as the inner member of the device of the invention. However, this should not be interpreted as limiting the disclosure.

**Fig. 5F** shows a first non-limiting example of a cutting tool configured according to some embodiments of the present disclosure. The figure describes a cutting tool **520E** which can minimize the radial attachment between the removed tissue and the cavity's inner surface to thereby minimize the effect of tearing of the tissue. This can be achieved by shaping of the cutting tool. As shown in the figure, a distal portion **504F** of the cutting tool is shaped and pressed towards rotation axis of the tool (passing at the center of the tool if the tool is symmetrical and isotropic). Pressing and shaping can be made by means of known techniques, such as swaging and spinning. In this case, the diameter of the cutting edge **522EF** at the distal end of the cutting tool is smaller than the inner diameter **505F** of the cavity inside the cutting tool. As the diameter of the cutting edge determines the diameter of the removed tissue, the cutting tool **520F** is expected to provide minimal or no attachment force on the removed tissue inside the tool and by this improve the trapping of the removed tissue, while minimizing tearing risk and preserving full shape of the removed tissue that matched a full open channel inside the tissue wall.

**Figs. 5G1 to 5G4** illustrate non-limiting exemplary cutting tools **520G1** (in Fig. 5G3) and **520G2** (in Fig. 5G4) and an exemplary process for producing the cutting tools (Figs. 5G1 and 5G2), according to some embodiments of the disclosure.

In **Fig. 5G1****,** a side view (cross section) of tool **520G** includes at a distal side **501G** a hollow cylinder **506G** having a cavity **507G** between uniform outer and inner diameters, **504G** and **505G** respectively, that extend along a longitudinal (rotation) axis **X1.** In **Fig. 5G2****,** a close-up view on the cylinder **206G** is shown. A distal portion **504G** is shaped with a predetermined pattern, e.g. by pressing, such that both the inner and outer diameters decrease towards the distal end **509G** of the hollow cylinder. As shown on the proximal end **511G** of the hollow cylinder, the original inner and outer diameters are about 0.17mm and 0.3mm respectively, and the modified inner and outer diameters at the distal end **509G** are about 0.13mm and 0.27mm respectively. The shaping of the distal portion can be done by, for example but not limited to, swaging and/or spinning techniques. The pattern of shaping can be linear, for example by tapering the distal portion to provide a substantially cylindrical frustum shape, or non-linear, e.g. by following a curved line such as parabolic or other similar pattern.

In a next step, a slice of the hollow cylinder's side wall is removed (to the right, in the figure) along the longitudinal axis, in the proximal direction, starting from the distal end **509G.** Depending on the slice depth (i.e. thickness), two exemplary cutting tools **520G1** and **520G2** are shown in **Figs. 5G3 and 5G4****.** A cutting edge **522EG** is formed at the distal end and the inner and outer diameters become almost equal, such as 0.18mm in **Fig. 5G3** and 0.16mm in **Fig. 5G4****.** Additional sharpening of the cutting edge, both from inside and outside directions results in that the cavity, at a distal side thereof, has a slightly smaller diameter than the cutting edge's diameter. Then, the inner diameter of the cavity increases continuously in the proximal direction (to the right in the figures) until the inner diameter of the cavity reaches the higher value **505G** of the proximal side of the hollow cylinder. Alternatively, the slicing of the inner surface of the cavity may provide a substantially constant inner cavity diameter. By this slicing step, no step (such as step 508E in Fig. 5E3) is present, and the cavity will have at least a length, in the longitudinal axis direction, equal to the original hollow cylinder's cavity length, thus providing receiving cavities longer than the limit of 0.5mm and wide enough to thereby minimize attachment of the tissue entering the cavity to the inner surface of the cavity. All at a micro level required for applications such as creating safe enough channels in the eye wall.

Other non-limiting examples of the inner member include devices as described in WO2013186779 and WO2015145444 both assigned to the assignee of the present invention.

As described above, the various movements of the outer and inner member of the device are performed either manually and/or by using a moving/movement mechanism. The outer member is configured for axial movement only, while the inner member is configured for both rotational and axial movements. Typically, the inner member's rotation is governed by an electrical motor connected to the proximal side of the inner member. This is not particularly described here, examples can be found in the above mentioned patent application PCT/IL2016/051063 assigned to the assignee of the present invention. In the following, a variety of moving/movement mechanisms are described.

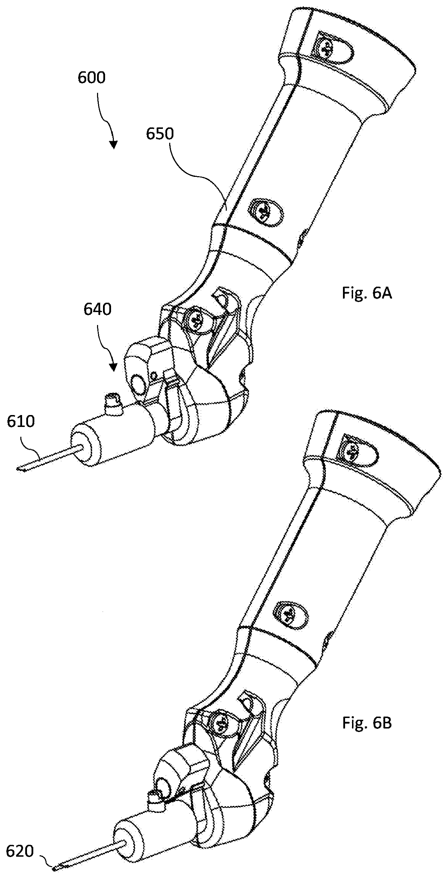

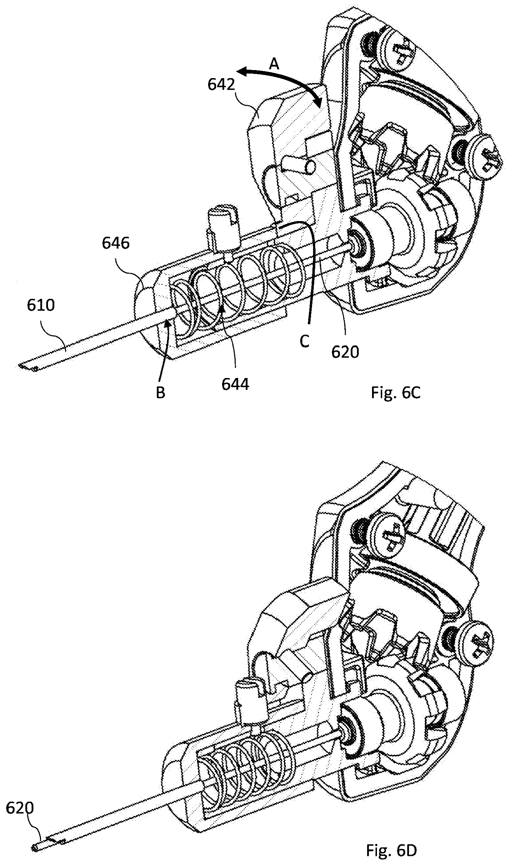

Reference is made to **Figs. 6A to 6D** illustrating a non-limiting example of a movement mechanism configured for manual movement of the device during operation. As shown, the device **600** includes outer member **610** and inner member **620** mounted on a handle **650** via a movement mechanism **640.** **Figs. 6A** and **6C** show the device during the positioning phase, i.e. when the outer member is moved forwardly (by manual pushing of the handle by the operating surgeon) to pierce first (preceding) and second (target) tissue layers, or to approach and stick into the target layer directly (such as in the Ab interno procedure). **Figs. 6B** and **6D** show the device during the channeling phase, i.e. when the inner member is rotated, by an electric motor (not shown), and advanced distally to cut and remove tissue, thereby leaving the channel in the target tissue layer.

The movement mechanism **640** includes a latch **642,** a spring **644,** and a housing **646.** As shown in **Fig. 6C****,** the latch is movable to the sides as illustrated by the arrow **A.** The outer member **610** is axially locked by being firmly attached to the housing at **B and** supported by the latch **642** at **C.** The spring **644** is slightly pre-compressed/relaxed during the positioning phase.

After pushing the device with the handle **650** inside the tissue until the first distal part of the outer member is stuck/anchored temporarily inside the target tissue layer, e.g. in the sclera, as described above, the operating surgeon turns the latch **642** to the left (or to the right) releasing the outer member **610** at **C,** thus enabling its retraction proximally. The surgeon switches the electrical motor to rotate the inner member and pushes distally with the handle **650** to expose the inner member **620** as in **Fig. 6D****.** The outer member **610** retracts and the spring **644** is compressed. The channel creation occurs once the surgeon pushes the device distally. While the compressed spring **644** tends to push the outer member **610** distally, the spring's constant is chosen to be too low to enable further penetration of the outer member to the sclera. Ergonomically, the surgeon can control over all features using a single finger while holding the handle.

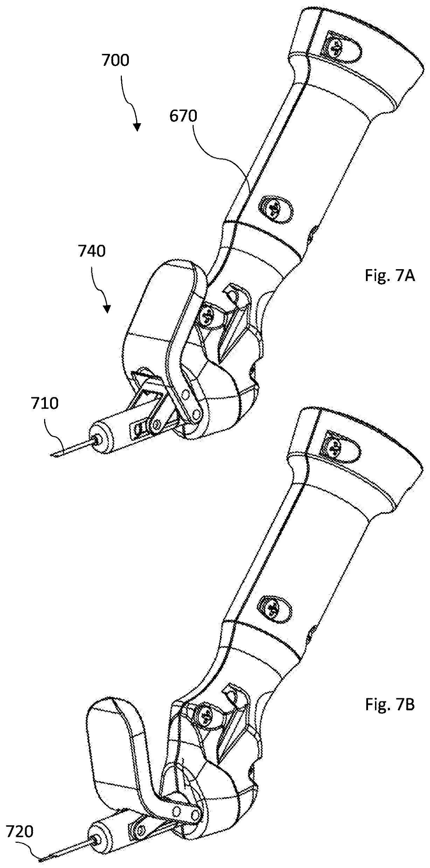

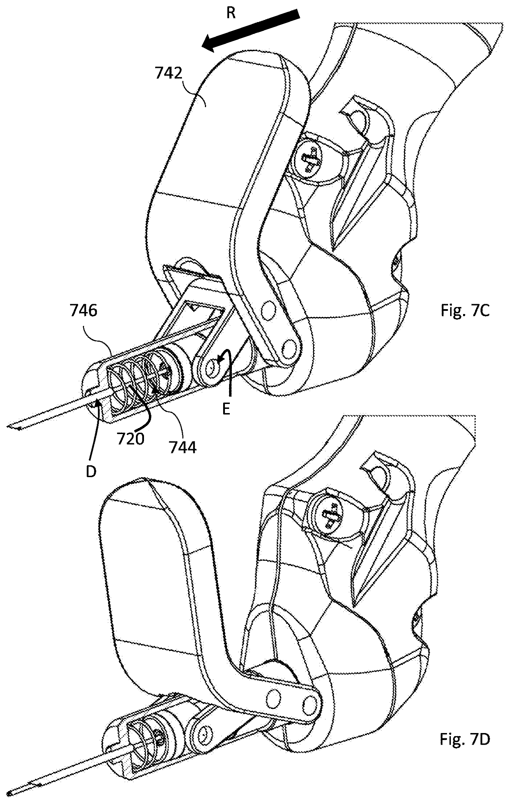

Reference is made to **Figs. 7A** to **7D** illustrating another non-limiting example of a movement mechanism configured for manual movement of the device, specifically the inner member, during operation. As shown, the device **700** includes outer member **710** and inner member **720** mounted on a handle **750** via a movement mechanism **740.** **Figs. 7A** and **7C** show the device during the positioning phase, i.e. when the outer member is moved forwardly (by manual pushing of the handle by the operating surgeon) to pierce one or more tissue layers and/or until being inserted and anchored in the target tissue layer. **Figs. 7B** and **7D** show the device during the channeling phase, i.e. when the inner member is rotated, by an electric motor, and advanced distally to create the channel in the second tissue layer.

The movement mechanism **740** is configured for controllably advancing the inner member (distally) by manual pushing movement. As shown in **Fig. 7C****,** the movement mechanism **740** includes a knob **742,** a spring **744,** and a housing **746.** The outer member **710** is axially locked by being fixedly attached to the housing at **D,** and as a result also to the handle **750,** such that when the operating surgeon pushes the handle **750** towards the tissue, the outer member moves in the axial direction and penetrates the tissue until it sticks in the sclera. The spring **744** is relaxed during the positioning phase.

The knob **742** is attached to the proximal side of the inner member **720** at **E,** such that they move together in the distal and proximal directions. During the channeling phase, the knob **742** is controllably pushed in the distal direction by the operating surgeon, as shown by arrow **R,** against the spring **744** causing it to compress. The inner member moves distally at the same rate by which the operating surgeon pushes the knob **742.** Upon releasing the knob **742,** a retraction movement occurs, the spring **744** relaxes and pulls the knob **742** as well as the inner member **720** proximally to the closed state as in **Fig. 7C****.** Additionally, though not specifically illustrated, the movement mechanism may include a latch configured to lock the knob **742** in the forward position, and only when the latch is released by the operating surgeon, the retraction movement occurs. As mentioned, the rotational movement of the inner member is controlled by an electrical motor which is not specifically described herein.

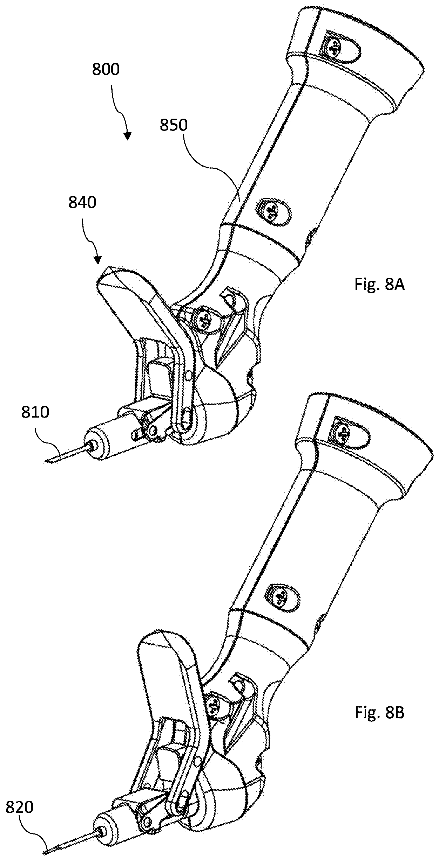

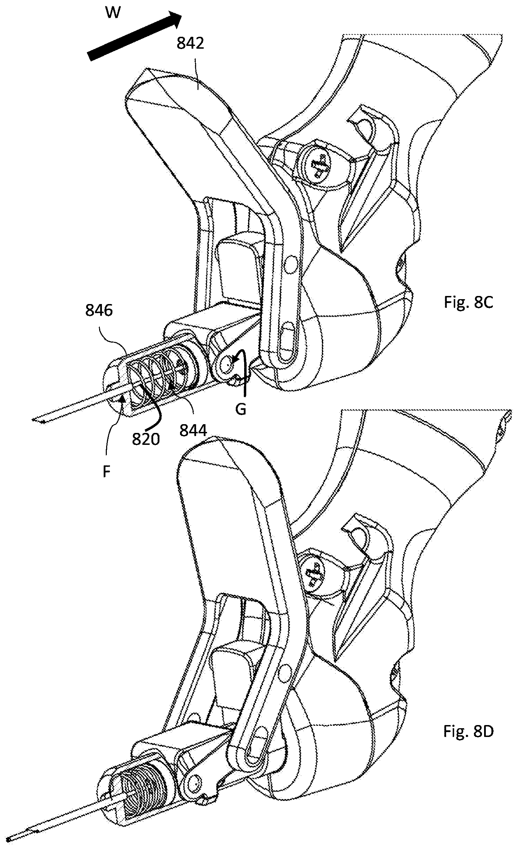

Reference is made to **Figs. 8A** to **8D** illustrating another non-limiting example of a movement mechanism configured for manual movement of the device, specifically the inner member, during operation. As shown, the device **800** includes outer member **810** and inner member **820** mounted on a handle **850** via a movement mechanism **840.** **Figs. 8A** and **8C** show the device during the positioning phase, i.e. when the outer member is moved forwardly (by manual pushing of the handle by the operating surgeon) to pierce one or more tissue layers and/or until being inserted and anchored in the target tissue layer. **Figs. 8B** and **8D** show the device during the channeling phase, i.e. when the inner member is rotated, by an electric motor (not shown), and advanced distally to create the channel in the target tissue layer.

The movement mechanism **840** is configured for controllably advancing the inner member (distally) by manual pulling movement. As shown in **Fig. 8C****,** the movement mechanism **840** includes a knob **842,** a spring **844,** and a housing **846.** The outer member **810** is axially locked by being fixedly attached to the housing at **F,** and as a result also to the handle **850,** such that when the operating surgeon pushes the handle **850** towards the tissue, the outer member moves in the axial direction and penetrates the tissue until it sticks in the sclera. The spring **844** is relaxed during the positioning phase.

The knob **842** is attached to the proximal side of the inner member **820** at **G,** such that they move together in the distal and proximal directions. During the channeling phase, the knob **842** is controllably pulled in the proximal direction by the operating surgeon, as shown by arrow **W,** such that **G** moves distally against the spring **844** causing it to compress. The inner member moves distally at the same rate by which the operating surgeon pulls the knob **842.** Upon releasing the knob **842,** it moves in the distal direction, the spring **844** relaxes and pushes **G** as well as the inner member **820** proximally to the closed state as in **Fig. 8C****.** Additionally, though not specifically illustrated, the movement mechanism may include a latch configured to lock the knob **842** in the backward position, and only when the latch is released by the operating surgeon, the retraction movement of the inner member occurs. As mentioned, the rotational movement of the inner member is controlled by an electrical motor which is not specifically described herein.

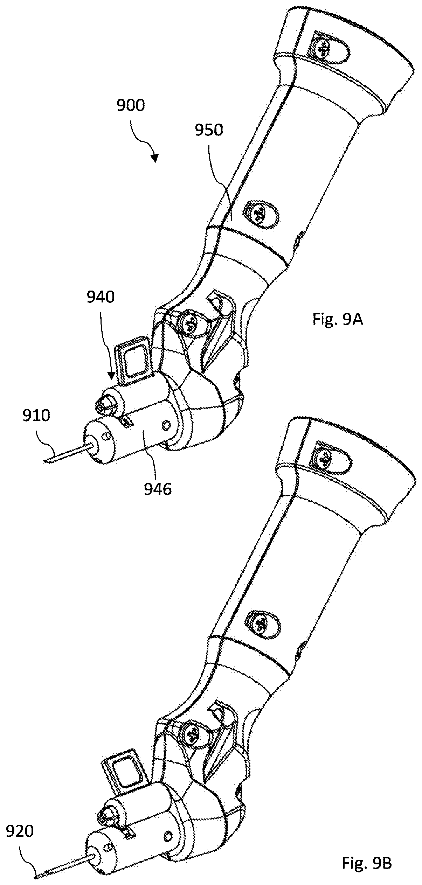

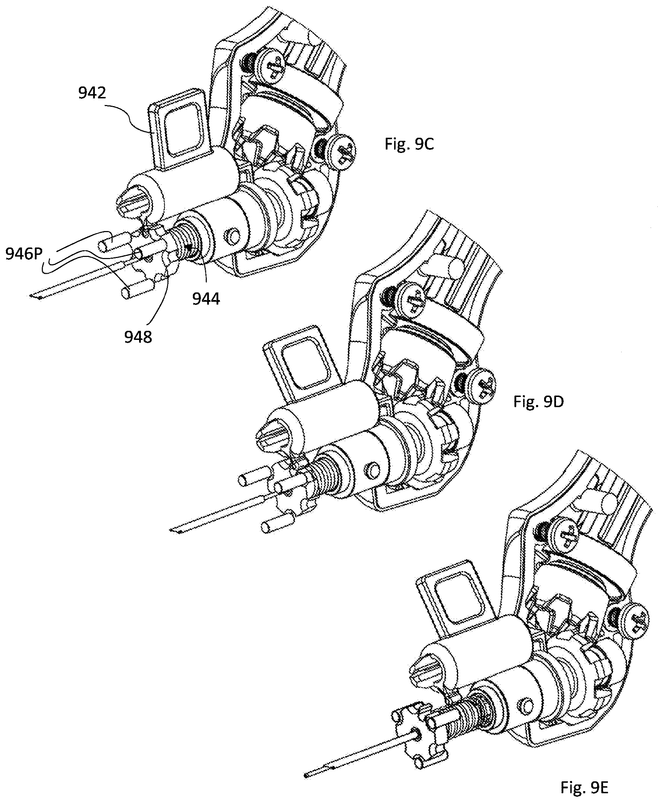

Reference is made to **Figs. 9A** to **9E** illustrating another non-limiting example of a movement mechanism configured for advancement of the inner member under constant or substantially constant force, e.g. 5 - 6N (with tolerance of about 1N). **Figs. 9A** and **9B** show the whole device **900.** As shown, the device **900** includes outer member **910** and inner member **920** mounted on a handle **950** via a movement mechanism **940.** **Figs. 9A****,** **9C** and **9D** show the device during the positioning phase, i.e. when the outer member is moved forwardly to pierce one or more tissue layers and/or until being inserted and anchored in the target tissue layer ( all by manual pushing of the handle by the operating surgeon). **Figs. 9B** and **9E** show the device during the channeling phase, i.e. when the inner member **920** is rotated, by an electric motor, and advanced distally to create the channel in the target tissue layer.

The movement mechanism **940** includes a knob **942,** a spring **944,** a floating disk **948** and a housing **946** including three pins **946P** firmly received therein in a spaced-apart relationship matching the floating disk's teeth. The outer member is permanently attached to the housing **946** such that it does not move relative to the handle **950,** and the outer member's axial movement is generated only by the operating surgeon by pushing the handle forwards and pulling it backwards.

During the positioning phase, as shown in **Fig. 9C****,** the spring **944** is compressed applying a distal pushing force on the floating disk **948.** However, the floating disk is kept stationary while the knob **942** is engaged with the floating disk **948** preventing from moving.

As shown in **Fig. 9D****,** after the outer member is inserted and anchored in the sclera, the operating surgeon turns on the electrical motor to cause rotation of the inner member, then turns the knob to the right or left and releases the floating disk **948.** Once the floating disk is released it turns and is pushed distally by the spring **944** which starts to relax, the floating disk engages with the pins **946P,** as shown in **Fig. 9E****.** The floating disk **948** is also axially attached to a base of the inner member, such that the floating disk's distal movement under the constant relaxing force of the spring **944** causes the rotating inner member to move distally under a constant force until the floating disk reaches the distal side of the housing **946** and the axial movement stops. Additionally, though not specifically illustrated, the movement mechanism may include a latch configured to lock the knob **942,** and only when the latch is released by the operating surgeon, the retraction movement of the inner member occurs.

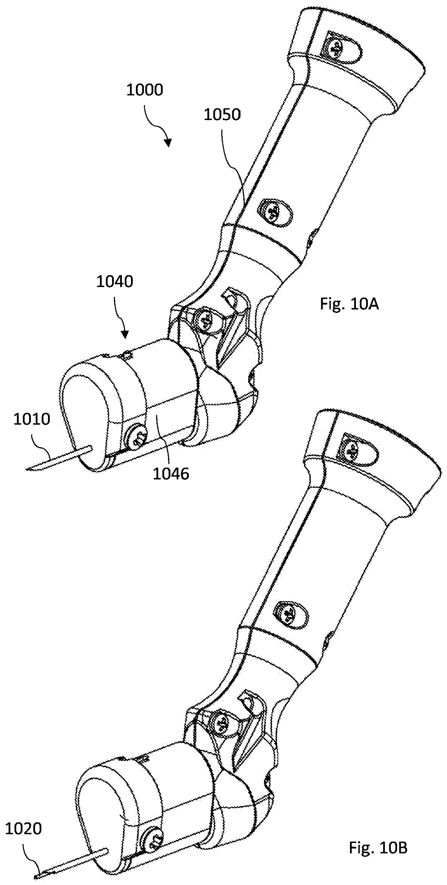

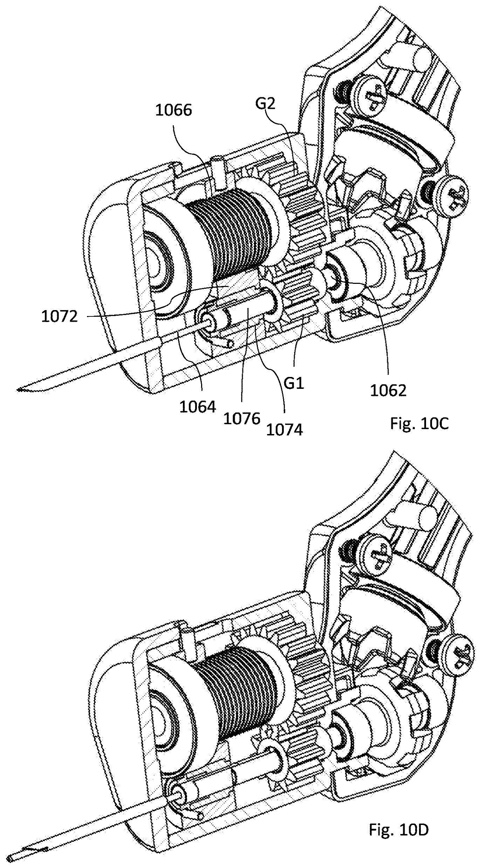

Reference is made to **Figs. 10A** to **10D** illustrating another non-limiting example of a movement mechanism configured for advancement of the inner member under constant rate. **Figs. 10A** and **10B** show the whole device **1000.** As shown, the device **1000** includes outer member **1010** and inner member **1020** mounted on a handle **1050** via a movement mechanism **1040.** **Figs. 10A** and **10C** show the device during the positioning phase, i.e. when the outer member is moved forwardly to pierce one or more tissue layers and/or until being inserted and anchored in the target tissue layer (by manual pushing of the handle by the operating surgeon). **Figs. 10B** and **10D** show the device during the channeling phase, i.e. when the inner member **1020** is rotated, by an electric motor, and advanced distally to create the channel in the target tissue layer.

The movement mechanism **1040** is configured for automatic rotation and advancement of the inner member. The rotation and advancement movements can be executed by the same or different motors. Additionally, the rate of the rotation and advancement movements can be the same or different, regardless of whether one or two separate motors are employed.

As shown in **Figs. 10C** and **10D****,** the movement mechanism **1040** includes two gears **G1 and G2.** Gear **G1** receives, at a first rate (e.g. 100 - 500 rounds per minute (RPM)), the rotational power delivered by the motor (which is not shown). Gear **G1** rotates with the inlet shaft **1062** which is constantly connected to Gear **G1** and is responsible for the rotational movement of the inner member **1020.** The inner member is connected to the inlet shaft **1062** via an outlet shaft **1064** which rotates together with the inlet shaft **1062** yet it can move axially with respect to the inlet shaft **1062.** Gear **G2** is engaged with gear **G1** such that it rotates according to a predetermined ratio between **G1** and **G2.** Gear **G2** is responsible for the axial movement of the inner member as shown in **Fig. 10D****.** Gear **G2** is connected constantly to a parallel shaft **1066** that has a built-in driving thread, as shown, such that the rotational movement of the Gear **G2** and the parallel shaft **1066** is translated to an axial movement, of a driving nut **1072,** via the built-in driving thread. The axial movement of the inner member is controlled by the housing's **1046** length along the axial direction. The driving nut **1072** is driven by the rotation of the parallel shaft **1066,** via the built-in thread, such that it moves to the distal direction and forces the outlet shaft **1064** distally with it. A bearing **1074** between the driving nut **1072** and outlet shaft **1064** enables the outlet shaft to rotate as it moves axially while a fork-like shaft **1076** enables the outlet shaft to continue turning along the axial distal travel.

## Claims

1. A medical device configured and operable for removing a defined shape of soft tissue from a target tissue layer in an eye thereby creating a matching channel with predetermined geometry and orientation between two side walls of the target tissue layer to enable drainage of fluid from the eye through the channel, the device comprising coaxial outer and inner elongated members extending along axis X;
said outer member comprising:
- a first distal part having a first open distal end and configured for sticking to said target tissue layer during forward axial movement of the outer member, and
- a first proximal elongated part housing the inner member;
said inner member being configured to be rotated and projected distally, along said axis X, through said first open distal end of the outer member, the inner member comprising:
- a second distal part having a second open distal end having a round cutting edge of a first diameter, the round cutting edge being configured to attach to said target tissue layer and cut said defined shape of the soft tissue from the target tissue layer while rotating and distally progressing, thereby creating said channel formed as a hole through the target tissue layer, and
- a chamber extending proximally inside the inner member from said second open distal end of the second distal part for a predetermined length, the chamber being configured to receive and retain said removed soft tissue in an intact form during said rotating and distally progressing of the inner member.

2. The device according to claim 1, wherein said first distal part of the outer member is configured for penetrating at least one other tissue layer preceding said target tissue layer during said forward axial movement, said first distal part comprising a tissue piercing tip at the first open distal end configured and operable to penetrate said at least one other tissue layer and said target tissue layer, and a proximal portion at a proximal side of the first distal part configured and operable to penetrate said at least one other tissue layer and to stop at said target tissue layer.

3. The device according to claim 2, wherein said first distal part has a predefined length such that , in use, said tissue piercing tip does not exit distally from said target tissue layer.

4. The device according to claim 2 or 3, wherein said proximal portion is a rim of said outer member, formed by cutting a section of wall of the outer member along said axis X.

5. The device according to any one of the preceding claims, having at least one of the following configurations:
a) said inner member is fixedly attached to and housed within said outer member during said forward axial movement of said outer member, and b) said outer member is configured to be manually moved during said forward axial movement until its said sticking to said target tissue layer.

6. The device according to any one of the preceding claims, having one of the following configurations:
a) said inner member is configured to, while rotating, be manually moved along said axis X to create the channel,
b) the device comprises a constant-force moving mechanism configured and operable to move said inner member, while rotating, along said axis X under a constant force,
c) the device comprises a constant rate moving mechanism configured and operable to move said inner member, while rotating, along said axis X with a constant rate, or
d) the device comprises an electric motor configured and operable for axially moving and/or rotating said inner member.

7. The device according to any one of the preceding claims, wherein said inner member comprises an elongated round body extending along the longitudinal axis X and having a uniform outer diameter at a proximal side thereof, said first diameter of the round cutting edge being smaller than said outer diameter and wherein said second distal part has a continuously decreasing outer diameter towards the second open distal end.

8. The device according to any one of the preceding claims, wherein said chamber has one of the following configurations:
a) the chamber has dimensions matching said predetermined shape of the soft tissue, said predetermined shape of the soft tissue is cylindrical and has a length of about 1.5mm and a diameter of between about 0.1mm and about 0.2mm,
b) the chamber has a length of at least the length of the removed soft tissue and has a chamber diameter smaller than said first diameter at a distal end of the chamber and which increases continuously towards a proximal end of the chamber, or
c) the chamber has a length of at least the length of the removed soft tissue and has a constant chamber diameter being equal to said first diameter, and wherein said first diameter is between about 0.1mm to about 0.2mm

9. The device according to any one of preceding claims , wherein said inner member comprises a tissue trapper located substantially in parallel to the chamber and configured to contribute to the trapping of the removed soft tissue during its removal.

10. The device according to claim 9, wherein said tissue trapper is a slit formed in wall of the body of the inner member along at least part of said chamber, said slit is formed by one of the following: a) tangential cutting of the wall of the body of inner member, said device thereby further comprising an outer chamber located between the inner and outer members, or b) radial cutting of the wall of the body of inner member.

11. The device according to any one of the preceding claims, wherein said rotating of said inner member comprises clockwise and anti-clockwise reciprocal movement.

12. The device according to any one of the preceding claims, wherein said first distal part of the outer member is formed by cutting the outer member in the direction of the axis X along a curved line chosen to provide smooth penetration, at a distal segment of the first distal part, and increasing resistance-to-progression force, at a proximal segment of the first distal part.

13. The device according to any one of the preceding claims, wherein said inner member is produced by the following steps:
- providing a member comprising at a distal side thereof a hollow cylinder having uniform outer and inner diameters and extending along at least said predetermined length, wherein said inner diameter is larger than said first diameter,
- shaping a distal portion of the hollow cylinder with a predetermined pattern such that both said inner and outer diameters decrease towards a distal end of the hollow cylinder and such that said first diameter is larger than said inner diameter and is smaller than said outer diameter at the distal end, and
- removing an inner slice of the hollow cylinder along said distal portion, such that the inner diameter at the distal end is substantially equal to said first diameter.

14. The device according to any one of the preceding claims wherein inner surface of said chamber is coated with a friction-lowering composition.

## Patentansprüche

1. Medizinische Vorrichtung, die dazu konfiguriert und betreibbar ist, eine definierte Form von Weichgewebe aus einer Zielgewebeschicht in einem Auge zu entfernen und dadurch einen entsprechenden Kanal mit vorbestimmter Geometrie und Ausrichtung zwischen zwei Seitenwänden der Zielgewebeschicht zu erzeugen, um einen Abfluss von Flüssigkeit aus dem Auge durch den Kanal zu ermöglichen, wobei die Vorrichtung koaxiale äußere und innere längliche Elemente umfasst, die sich entlang einer Achse X erstrecken;
wobei das äußere Element Folgendes umfasst:
- einen ersten distalen Teil, der ein erstes offenes distales Ende aufweist und dazu konfiguriert ist, an der Zielgewebeschicht während einer axialen Vorwärtsbewegung des äußeren Elements zu haften, und
- einen ersten proximalen, länglichen Teil, der das innere Element aufnimmt;
wobei das innere Element dazu konfiguriert ist, gedreht und entlang der Achse X durch das erste offene distale Ende des äußeren Elements distal vorgeschoben zu werden, wobei das innere Element Folgendes umfasst:
- einen zweiten distalen Teil, der ein zweites offenes distales Ende aufweist, das eine runde Schneidkante mit einem ersten Durchmesser aufweist, wobei die runde Schneidkante dazu konfiguriert ist, an der Zielgewebeschicht anzuliegen und die definierte Form des Weichgewebes aus der Zielgewebeschicht herauszuschneiden, während sie sich dreht und distal vorrückt, wodurch der als ein Loch durch die Zielgewebeschicht ausgebildete Kanal erzeugt wird, und
- eine Kammer, die sich innerhalb des inneren Elements von dem zweiten offenen distalen Ende des zweiten distalen Teils über eine vorbestimmte Länge proximal erstreckt, wobei die Kammer dazu konfiguriert ist, das entfernte Weichgewebe in einer intakten Form während des Drehens und distalen Vorrückens des inneren Elements aufzunehmen und aufzubewahren.

2. Vorrichtung nach Anspruch 1, wobei der erste distale Teil des äußeren Elements dazu konfiguriert ist, mindestens eine weitere Gewebeschicht, die der Zielgewebeschicht vorgelagert ist, während der axialen Vorwärtsbewegung zu durchdringen, wobei der erste distale Teil eine Gewebedurchstichspitze an dem ersten offenen distalen Ende, die dazu konfiguriert und betreibbar ist, die mindestens eine weitere Gewebeschicht und die Zielgewebeschicht zu durchdringen, und einen proximalen Abschnitt an einer proximalen Seite des ersten distalen Teils, der dazu konfiguriert und betreibbar ist, die mindestens eine weitere Gewebeschicht zu durchdringen und bei der Zielgewebeschicht anzuhalten, umfasst.

3. Vorrichtung nach Anspruch 2, wobei der erste distale Teil eine vordefinierte Länge aufweist, sodass bei Verwendung die Gewebedurchstichspitze nicht distal aus der Zielgewebeschicht herausragt.

4. Vorrichtung nach Anspruch 2 oder 3, wobei der proximale Abschnitt ein Rand des äußeren Elements ist, der durch Herausschneiden eines Wandbereichs des äußeren Elements entlang der Achse X ausgebildet wird.

5. Vorrichtung nach einem der vorhergehenden Ansprüche, die mindestens eine der folgenden Konfigurationen aufweist:
a) während der axialen Vorwärtsbewegung des äußeren Elements liegt das innere Element fest an dem äußeren Element an und ist darin aufgenommen, und b) das äußere Element ist dazu konfiguriert, während der axialen Vorwärtsbewegung, bis zu seinem Haften an der Zielgewebeschicht manuell bewegt zu werden.

6. Vorrichtung nach einem der vorhergehenden Ansprüche, die eine der folgenden Konfigurationen aufweist:
a) das innere Element ist dazu konfiguriert, während es sich dreht, manuell entlang der Achse X bewegt zu werden, um den Kanal zu erzeugen,
b) die Vorrichtung umfasst einen Bewegungsmechanismus mit konstanter Kraft, der dazu konfiguriert und betreibbar ist, das innere Element, während es sich dreht, entlang der Achse X unter einer konstanten Kraft zu bewegen,
c) die Vorrichtung umfasst einen Bewegungsmechanismus mit konstanter Geschwindigkeit, der dazu konfiguriert und betreibbar ist, das innere Element, während es sich dreht, entlang der Achse X mit einer konstanten Geschwindigkeit zu bewegen, oder
d) die Vorrichtung umfasst einen elektrischen Motor, der dazu konfiguriert und betreibbar ist, das innere Element axial zu bewegen und/oder zu drehen.

7. Vorrichtung nach einem der vorhergehenden Ansprüche, wobei das innere Element einen länglichen, runden Körper umfasst, der sich entlang der Längsachse X erstreckt und einen gleichmäßigen Außendurchmesser an einer proximalen Seite aufweist, wobei der erste Durchmesser der runden Schneidkante kleiner ist als der Außendurchmesser, und wobei der zweite distale Teil einen zu dem zweiten offenen distalen Ende hin kontinuierlich abnehmenden Außendurchmesser aufweist.

8. Vorrichtung nach einem der vorhergehenden Ansprüche, wobei die Kammer eine der folgenden Konfigurationen aufweist:
a) die Kammer weist Abmessungen auf, die der vorbestimmten Form des Weichgewebes entsprechen, die vorbestimmte Form des Weichgewebes ist zylindrisch und weist eine Länge von etwa 1,5 mm und einen Durchmesser zwischen etwa 0,1 mm und etwa 0,2 mm auf,
b) die Kammer weist eine Länge auf, die mindestens der Länge des entfernten Weichgewebes entspricht, und weist einen Kammerdurchmesser auf, der kleiner als der erste Durchmesser an einem distalen Ende der Kammer ist und der zu einem proximalen Ende der Kammer hin kontinuierlich zunimmt, oder
c) die Kammer weist eine Länge auf, die mindestens der Länge des entfernten Weichgewebes entspricht, und weist einen konstanten Kammerdurchmesser auf, der gleich dem ersten Durchmesser ist, und wobei der erste Durchmesser zwischen etwa 0,1 mm bis etwa 0,2 mm liegt

9. Vorrichtung nach einem der vorhergehenden Ansprüche, wobei das innere Element eine Gewebeauffangeinrichtung umfasst, die im Wesentlichen parallel zu der Kammer angeordnet ist und dazu konfiguriert ist, zu dem Auffangen des entfernten Weichgewebes während dessen Entfernung beizutragen.

10. Vorrichtung nach Anspruch 9, wobei die Gewebeauffangeinrichtung ein Schlitz ist, der in einer Wand des Körpers des inneren Elements entlang mindestens eines Teils der Kammer ausgebildet ist, wobei der Schlitz durch eines der Folgenden ausgebildet ist: a) tangentiales Einschneiden der Wand des Körpers des inneren Elements, wobei die Vorrichtung dadurch ferner eine äußere Kammer, die zwischen dem inneren Element und dem äußeren Element angeordnet ist, umfasst, oder b) radiales Einschneiden der Wand des Körpers des inneren Elements.

11. Vorrichtung nach einem der vorhergehenden Ansprüche, wobei das Drehen des inneren Elements eine Hin- und Her-Bewegung im Uhrzeigersinn und gegen den Uhrzeigersinn umfasst.

12. Vorrichtung nach einem der vorhergehenden Ansprüche, wobei der erste distale Teil des äußeren Elements durch Einschneiden des äußeren Elements in der Richtung der Achse X entlang einer gekrümmten Linie ausgebildet wird, die so gewählt ist, dass sie ein sanftes Durchdringen an einem distalen Segment des ersten distalen Teils und eine steigende Widerstandskraft gegen das Vorrücken an einem proximalen Segment des ersten distalen Teils bereitstellt.

13. Vorrichtung nach einem der vorhergehenden Ansprüche, wobei das innere Element durch die folgenden Schritte erzeugt wird:
- Bereitstellen eines Elements, das an seiner distalen Seite einen Hohlzylinder umfasst, der gleichmäßige Außen- und Innendurchmesser aufweist und sich über mindestens die vorbestimmte Länge erstreckt, wobei der Innendurchmesser größer ist als der erste Durchmesser,
- Formen eines distalen Abschnitts des Hohlzylinders mit einem vorbestimmten Muster, derart, dass sowohl der Innendurchmesser als auch der Außendurchmesser zu einem distalen Ende des Hohlzylinders hin abnimmt und derart, dass der erste Durchmesser größer ist als der Innendurchmesser und kleiner ist als der Außendurchmesser an dem distalen Ende, und
- Entfernen einer inneren Schicht des Hohlzylinders entlang des distalen Abschnitts, derart, dass der Innendurchmesser an dem distalen Ende im Wesentlichen gleich dem ersten Durchmesser ist.

14. Vorrichtung nach einem der vorhergehenden Ansprüche, wobei eine Innenfläche der Kammer mit einer reibungsmindernden Zusammensetzung beschichtet ist.

## Revendications

1. Dispositif médical configuré et utilisable pour le retrait d'une forme définie de tissu mou à partir d'une couche de tissu cible dans un œil, créant ainsi un canal correspondant avec une géométrie et une orientation prédéterminées entre deux parois latérales de la couche de tissu cible pour permettre le drainage de fluide à partir de l'œil à travers le canal, le dispositif comprenant des éléments allongés externe et interne coaxiaux s'étendant le long d'un axe X ;
ledit élément externe comprenant :
- une première partie distale ayant une première extrémité distale ouverte et configurée pour le collage à ladite couche de tissu cible pendant un mouvement axial vers l'avant de l'élément externe, et
- une première partie allongée proximale logeant l'élément interne ;
ledit élément interne étant configuré pour être mis en rotation et projeté de manière distale, le long dudit axe X, à travers ladite première extrémité distale ouverte de l'élément externe, l'élément interne comprenant :
- une seconde partie distale ayant une seconde extrémité distale ouverte ayant un bord de coupe rond d'un premier diamètre, le bord de coupe rond étant configuré pour se fixer à ladite couche de tissu cible et couper ladite forme définie du tissu mou à partir de la couche de tissu cible tout en tournant et en progressant de manière distale, créant ainsi ledit canal formé comme un trou à travers la couche de tissu cible, et
- une chambre s'étendant de manière proximale à l'intérieur de l'élément interne à partir de ladite seconde extrémité distale ouverte de la seconde partie distale sur une longueur prédéterminée, la chambre étant configurée pour la réception et la retenue dudit tissu mou retiré dans une forme intacte pendant ladite rotation et ladite progression distale de l'élément interne.

2. Dispositif selon la revendication 1, dans lequel ladite première partie distale de l'élément externe est configurée pour la pénétration d'au moins une autre couche de tissu précédant ladite couche de tissu cible pendant ledit mouvement axial vers l'avant, ladite première partie distale comprenant une pointe de perçage de tissu au niveau de la première extrémité distale ouverte configurée et utilisable pour la pénétration de ladite au moins une autre couche de tissu et de ladite couche de tissu cible, et une portion proximale au niveau d'un côté proximal de la première partie distale configurée et utilisable pour la pénétration de ladite au moins une autre couche de tissu et pour l'arrêt au niveau de ladite couche de tissu cible.

3. Dispositif selon la revendication 2, dans lequel ladite première partie distale a une longueur prédéfinie de sorte que, en cours d'utilisation,
ladite pointe de perçage de tissu ne sort pas de manière distale de ladite couche de tissu cible.

4. Dispositif selon la revendication 2 ou 3, dans lequel ladite portion proximale est un rebord dudit élément externe, formé par la découpe d'une section de paroi de l'élément externe le long dudit axe X.

5. Dispositif selon l'une quelconque des revendications précédentes, ayant au moins l'une des configurations suivantes :
a) ledit élément interne est fixé de manière inamovible à et logé à l'intérieur dudit élément externe pendant ledit mouvement axial vers l'avant dudit élément externe, et b) ledit élément externe est configuré pour être
déplacé manuellement pendant ledit mouvement axial vers l'avant jusqu'à son dit collage à ladite couche de tissu cible.

6. Dispositif selon l'une quelconque des revendications précédentes, ayant l'une des configurations suivantes :
a) ledit élément interne est configuré pour, tout en tournant, être déplacé manuellement le long dudit axe X pour créer le canal,
b) le dispositif comprend un mécanisme de déplacement à force constante configuré et utilisable pour le déplacement dudit élément interne, tout en tournant, le long dudit axe X sous une force constante,
c) le dispositif comprend un mécanisme de déplacement à vitesse constante configuré et utilisable pour le déplacement dudit élément interne, tout en tournant, le long dudit axe X à une vitesse constante, ou
d) le dispositif comprend un moteur électrique configuré et utilisable pour le déplacement axial et/ou la rotation dudit élément interne.

7. Dispositif selon l'une quelconque des revendications précédentes, dans lequel ledit élément interne comprend un corps rond allongé s'étendant le long de l'axe longitudinal X et ayant un diamètre externe uniforme au niveau d'un côté proximal de celui-ci, ledit premier diamètre du bord de coupe rond étant inférieur audit diamètre externe et dans lequel
ladite seconde partie distale a un diamètre externe diminuant de façon continue en direction de la seconde extrémité distale ouverte.

8. Dispositif selon l'une quelconque des revendications précédentes, dans lequel ladite chambre a l'une des configurations suivantes :
a) la chambre a des dimensions correspondant à ladite forme prédéterminée du tissu mou, ladite forme prédéterminée du tissu mou est cylindrique et a une longueur d'environ 1,5 mm et un diamètre compris entre environ 0,1 mm et environ 0,2 mm,
b) la chambre a une longueur d'au moins la longueur du tissu mou retiré et a un diamètre de chambre inférieur audit premier diamètre au niveau d'une extrémité distale de la chambre et qui augmente de façon continue en direction d'une extrémité proximale de la chambre, ou
c) la chambre a une longueur d'au moins la longueur du tissu mou retiré et a un diamètre de chambre constant qui est égal audit premier diamètre, et dans lequel ledit premier diamètre est compris entre environ 0,1 mm à environ 0,2 mm.

9. Dispositif selon l'une quelconque des revendications précédentes, dans lequel ledit
élément interne comprend un dispositif de piégeage de tissu situé de manière sensiblement parallèle à la chambre et configuré pour contribuer au piégeage du tissu mou retiré pendant son retrait.

10. Dispositif selon la revendication 9, dans lequel ledit dispositif de piégeage de tissu est une fente formée dans une paroi du corps de l'élément interne le long d'au moins une partie de ladite chambre, ladite fente est formée par l'une de ce qui suit : a) la découpe tangentielle de la paroi du corps de l'élément interne, ledit dispositif comprenant ainsi en outre une chambre externe située entre les éléments interne et externe, ou b) la découpe radiale de la paroi du corps de l'élément interne.

11. Dispositif selon l'une quelconque des revendications précédentes, dans lequel ladite rotation dudit élément interne comprend un mouvement de va-et-vient dans le sens des aiguilles d'une montre et dans le sens inverse des aiguilles d'une montre.

12. Dispositif selon l'une quelconque des revendications précédentes, dans lequel ladite première partie distale de l'élément externe est formée par la découpe de l'élément externe dans la direction de l'axe X le long d'une ligne incurvée choisie pour la fourniture d'une pénétration douce, au niveau d'un segment distal de la première partie distale, et d'une force de résistance à la progression croissante, au niveau d'un segment proximal de la première partie distale.

13. Dispositif selon l'une quelconque des revendications précédentes, dans lequel ledit élément interne est produit par les étapes suivantes :
- la fourniture d'un élément comprenant, au niveau d'un côté distal de celui-ci, un cylindre creux ayant des diamètres externe et interne uniformes et s'étendant le long d'au moins ladite longueur prédéterminée, dans lequel ledit diamètre interne est supérieur audit premier diamètre,
- le façonnage d'une portion distale du cylindre creux avec un motif prédéterminé de sorte qu'à la fois lesdits diamètres interne et externe diminuent en direction d'une extrémité distale du cylindre creux et de sorte que ledit premier diamètre est supérieur audit diamètre interne et est inférieur audit diamètre externe au niveau de l'extrémité distale, et
- le retrait d'une tranche interne du cylindre creux le long de ladite portion distale, de sorte que le diamètre interne au niveau de l'extrémité distale est sensiblement égal audit premier diamètre.

14. Dispositif selon l'une quelconque des revendications précédentes, dans lequel une surface interne de ladite chambre est revêtue d'une composition abaissant le frottement.
